# EUROPEAN PATENT SPECIFICATION

(11) **EP 0 739 901 B1**
(45) Date of publication and mention of the grant of the patent: **19.12.2001**
(21) Application number: 96106545.5
(22) Date of filing: 13.04.1989
(51) Int. Cl.: C07H 21/00, A61K 31/70, C07J 51/00, A61K 31/58

(54) **Novel oligoribonucleotide derivatives and application thereof to antiviral agents**
Oligoribonukleotid-Derivate und Verwendung davon als Antiviral Arzneimittel
Dérivés d'oligoribonucléotides et leur application comme agents antiviraux

(30) Priority: 27.04.1988 JP 10494388; 06.06.1988 JP 13896688; 06.07.1988 JP 16814288; 12.09.1988 JP 22788788; 22.09.1988 JP 23848188; 02.02.1989 JP 2437289; 16.03.1989 JP 6405889
(43) Date of publication of application: 30.10.1996
(62) Divisional of application: 89303700.2
(73) Proprietor: ISIS PHARMACEUTICALS, INC., Carlsbad, CA 92008 (US)
(72) Inventor: Shibahara, Susumu, c/o Central Research Lab., Kawasaki-shi, Kanagawa-ken (JP); Morisawa, Hirokazu, c/o Central Research Lab., Kawasaki-shi, Kanagawa-ken (JP); Nakajima, Hideki, Ube-shi, Yamaguchi-ken (JP); Yamamoto, Naoki, Ube-shi, Yamaguchi-ken (JP); Mukai, Sachiko, c/o Central Research Lab., Kawasaki-shi, Kanagawa-ken (JP)
(74) Representative: Bond, Bentley George

(56) References cited:
- EP-A- 0 169 775
- VIROLOGY, vol. 42, no. 2, October 1970, ORLANDO, FL, USA, pages 421-428, XP002012066 E. DE CLERCO ET AL: "The Anti-Viral Activity of Thiophosphate-Substituted Polyribonucleotides "In Vitro" and "In Vivo""
- CHEMICAL ABSTRACTS, vol. 106, no. 19, 11 May 1987 Columbus, Ohio, US; abstract no. 156829, E. OTSUKA ET AL: "2'-O-Methyl RNA" page 726; column 2; XP002012075 & JP-A-61 291 595 (KOKAI TOKKYO KOHO) 22 December 1986
- NATURE, vol. 313, no. 6000, January 1985, LONDON GB, pages 277-284, XP002012067 L. RATNER ET AL: "Complete Nucleotide Sequence of the AIDS Virus, HTLV-III"
- BIOCHEMISTRY., vol. 18, no. 19, 18 September 1979, EASTON, PA US, pages 4116-4120, XP002012068 D. YEE ET AL: "Mechanistic Studies on Deoxyribonucleic Acid Dependent Ribonucleic Acid Polymerase from Escherichia coli Using Phosphorothioate Analogues. 1. Initiation and Pyrophosphate Exchange Reactions"
- BIOCHEMISTRY., vol. 21, no. 23, 9 November 1982, EASTON, PA US, pages 5877-5885, XP002012069 F. R. BRYANT ET AL: "Phosphorothioate Substrates for T4 RNA Ligase"
- NUCLEIC ACIDS RESEARCH., vol. 16, no. 4, 25 February 1988, ARLINGTON, VIRGINIA US, pages 1577-1591, XP002012070 A. W. NICHOLSON ET AL: "Accurate "In Vivo" Cleavage by RNAse III of Phosphorothioate-Substituted RNA Processing Signals in Bacteriophage T7 early mRNA"
- JOURNAL OF THE AMERICAN CHEMICAL SOCIETY., vol. 103, no. 3, 11 February 1981, GASTON, PA US, pages 696-697, XP002012071 F. R. BRYANT ET AL: "On the Mechanism of T4 RNA Ligase"
- NUCLEIC ACIDS RESEARCH SYMPOSIUM SERIES - SEVENTH SYMPOSIUM ON THE CHEMISTRY OF NUCLEIC ACID COMPONENTS HELD AT BECHYNE CASTLE, CZECHOSLOVAKIA, FROM 30-08-87 - 05-09-87, no. 18, 1987, IRL PRESS LTD, OXFORD GB, pages 277-280, XP002012072 C. DEENEY ET AL: "Self Splicing of Tetrahymena rRNA Can Proceed with Phosphorothioate Substituent at the Splice Sites"
- PROCEEDINGS OF THE NATIONAL ACADEMY OF SCIENCES OF USA, vol. 84, no. 21, November 1987, WASHINGTON US, pages 7706-7710, XP002012073 M. MATSUKURA ET AL: "Phosphorothioate Analogs of Oligodeoxynucleotides: Inhibitors of Replication and Cytopathic Effects of Human Immunodeficiency Virus"
- NUCLEIC ACIDS RESEARCH., vol. 17, no. 1, 11 January 1989, ARLINGTON, VIRGINIA US, pages 239-252, XP002012074 S. SHIBAHARA ET AL: "Inhibition of Human Immunodeficiency Virus (HIV-1) Replication by Synthetic Oligo-RNA Derivatives"

## Description

The present invention relates to novel compounds, namely ribooligonucleotide phosphorothioate derivatives which can inhibit proliferation of AIDS virus (HIV-1, HIV-2, etc.) causing AIDS (acquired immunodeficiency syndrome), to make AIDS virus avirulent, novel intermediates for producing the same and the application of these derivatives to drugs, for example, antiviral agents such as anti-AIDS drugs.

AIDS (acquired immunodeficiency syndrome) was found in 1981 and it has been clarified that the cause lies in HIV (human immunodeficiency virus, hereafter simply referred to as AIDS virus) belonging to the family retro virus. The number of patients shows a yearly increase all over the world. According to the WHO's report, the number of patients exceeded 110,000 in the world as of August 31, 1988. In Japan, 90 patients are reported (as of August 31, 1988). Furthermore, if carriers infected with the virus but developing no AIDS yet are included, the number of patients will be more than 10,000,000 (estimated by WHO) in the world and 1048 in Japan (reported by the Ministry of Health and Welfare, as of August 31, 1988). A drug for completely curing AIDS that is a serious disease has not been found so far. 3'-Deoxy-3'-azidothymidine (hereafter simply referred to as "AZT") has been used in the clinical field as a drug for retarding the progress of AIDS and improving the clinical syndrome of the patient. However, side effect such as bone marrow impairment, etc. associated with its effectiveness are reported (cf. The New England Journal of Medicine, 317, 192-197, 1987). The mechanism of AZT is based on inhibition of the activity of reverse transcriptase possessed by the retro virus itself which is utilized, after invasion of the virus into host cells, to transcribe its genomic RNA onto DNA (Mitsuya, H., et al., Proc. Natl. Acad. Sci. USA, 82, 7096-7100, 1985) and it is thus probable that the mechanism would be related to the side effects.

On the other hand, it has been recently reported that phosphorothioate derivatives of deoxyoligonucelotides exhibit an anti-AIDS viral activity (cf. Proc. Natl. Acad. Sci. USA, 84, 7706-7710, 1987).

The AIDS virus has extremely frequent variations among its strains, in respect of its envelope protein. Therefore, it is extremely difficult to develop an effective vaccine and chemotherapeutic agents as described above are desired. However, nucleocido derivatives such as AZT conventionally used as chemotherapeutic agents exhibit anti-AIDS viral activity on one hand but on the other hand, involve serious side effects. Accordingly, it has been desired to develop drugs having minimized side effects and high practicability. For achieving the purpose, deoxyoligonucleotide phosphorothioate derivatives are at the stage of basic studies but it is desired to develop drugs having a more potent activity and provide such drugs at low costs.

As a result of extensive investigations to solve the foregoing problems, the present inventors have found that newly produced 2'-O-methyloligoribonucleotide derivatives and oligoribonucleotide derivatives could inhibit infection and proliferation of the AIDS virus to and in host cells and are applicable as drugs for treating AIDS, and have accomplished the present invention based on this finding.

Thus, the present invention is directed to:
novel oligoribonucleotide derivatives represented by general formula (1) described below, and anti-AIDS viral agents comprising the oligoribonucleotide derivatives as the effective ingredient;
novel oligoribonucleotide derivatives represented by general formula (4) described below and anti-AIDS viral compositions comprising the derivatives as the effective ingredient.

### General formula (I)

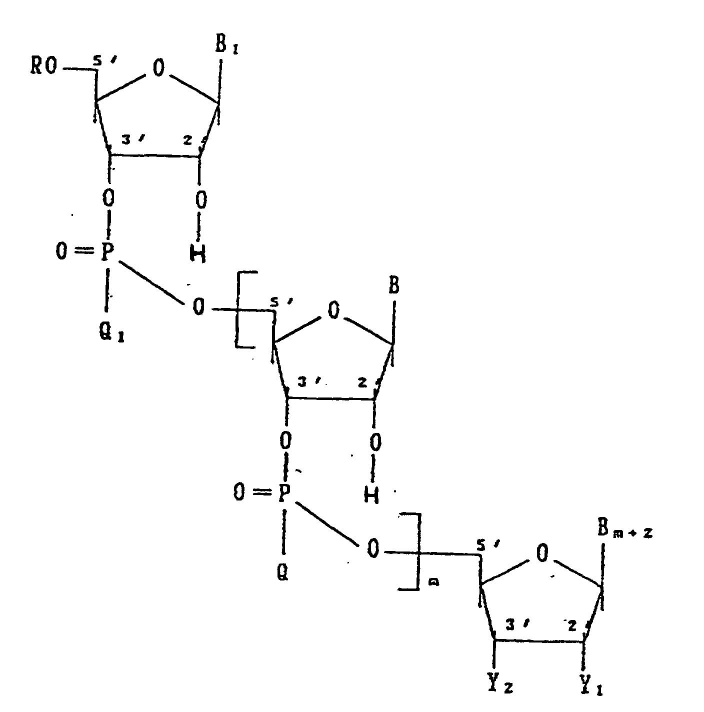

wherein:
m is an integer of 4 to 50;
B represents B₂, B₃, ..... B₍ₘ₊₁₎ and, B₁ through B₍ₘ₊₂₎, which may be the same or different, each represents any one of hypoxanthine-9-yl, guanine-9-yl, adenine-9-yl, cytosine-l-yl, uracil-1-yl and thymine-1-yl;
Q represents Q₂, Q₃ ..... Q₍ₘ₊₁₎ and, Q₁ through Q₍ₘ₊₁₎, independently represents any one of a thio anion, an oxo anion, an alkyl group, an aryloxy group, an alkylamino group, an arylamino group, an alkylthio group and an arylthio group (provided that at least one of Q₁ to Q₍ₘ₊₁₎ represents a thio anion);
R represents a hydrogen atom, an acyl group having 1 to 20 carbon atoms, a phosphoryl group which may optionally have a substituent, a thiophosphoryl group which may optionally have a substituent or an alkylaminophosphoryl group which may optionally have a substituent;
Y₁ and Y₂ independently represent a methoxy group, an acyloxy group having 1 to 20 carbon atoms, a hydrogen atom, hydroxyl group, an alkyloxy group which may optionally have a substituent, a phosphoryloxy group which may optionally have a substituent, a thiophosphoryloxy group which may optionally have a substituent and an alkylaminophosphoryloxy group which may optionally have a substituent. Herein the alkyl represents a straight or branched chain hydrocarbon residue having 1 to 30 carbon atoms and the aryl represents a hydrocarbon residue having 6 to 30 carbon atoms including phenyl group.

Further in formula (1) described above, examples of the substituent on R, Y₁ and Y₂ include cyanoethyl group, chlorophenyl group, monophosphoryl group, pyrophosphoryl group, a hydrocarbon residue having 1 to 20 carbon atoms, cholesteryl group and a group shown by J-(K-O-L)̵_{E}, which may be the same or different: provided that J represents: 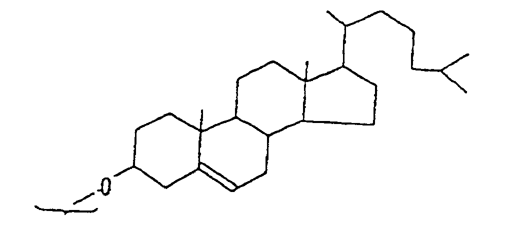 hydroxyl group or any one of the oligoribonucleotidyl group defined for the structural formula of said derivative according to claim 1, from which any one of RO, Y₁ and Y₂ is removed;
K represents any one of phosphoryl group, thiophosphoryl group and an alkylaminophosphoryl group having 1 to 10 carbon atoms;
L represents a hydrocarbon residue having 1 to 20 carbon atoms; and,
E represents an integer of 1 to 10.

The nucleotide sequence may not necessarily be complementary to the sequence of genomic RNA of AIDS virus or DNA integrated into chromosome.

In the formula described above, B₁ through B₍ₘ₊₂₎ and Q₁ through Q₍ₘ₊₁₎ are shown from the 5'-end in the order of 1, 2, 3, 4, ... (m+1). For example, when m is 2, B₁ to B₍ₘ₊₂₎ are, in the order from the 5'-end, B₁, B₂, B₃, B₄; Q₁ to Q₍ₘ₊₁₎ are, in the order from the 5'-end, Q₁, Q₂, Q₃; and the following structural formula is presented;

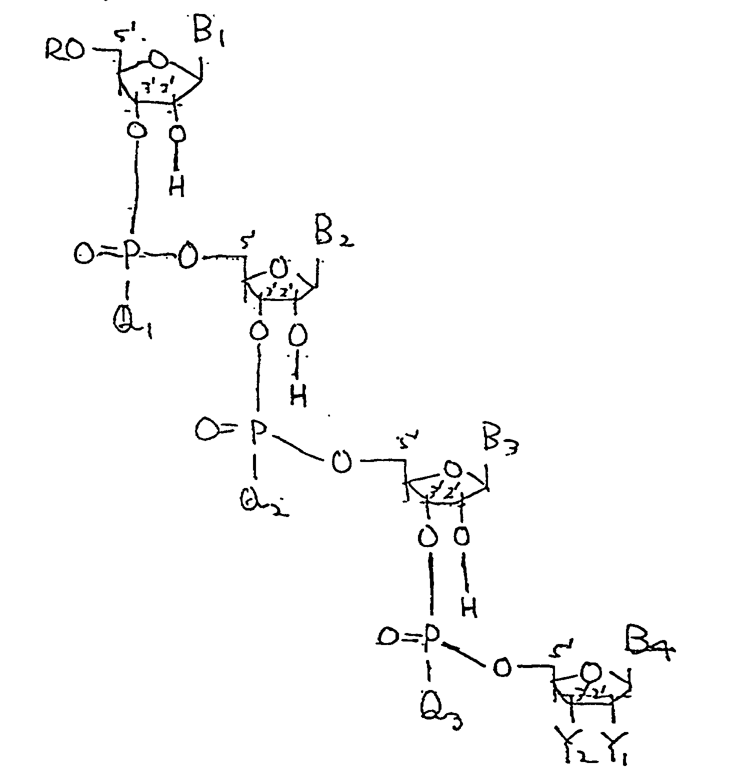

### General formula (4)

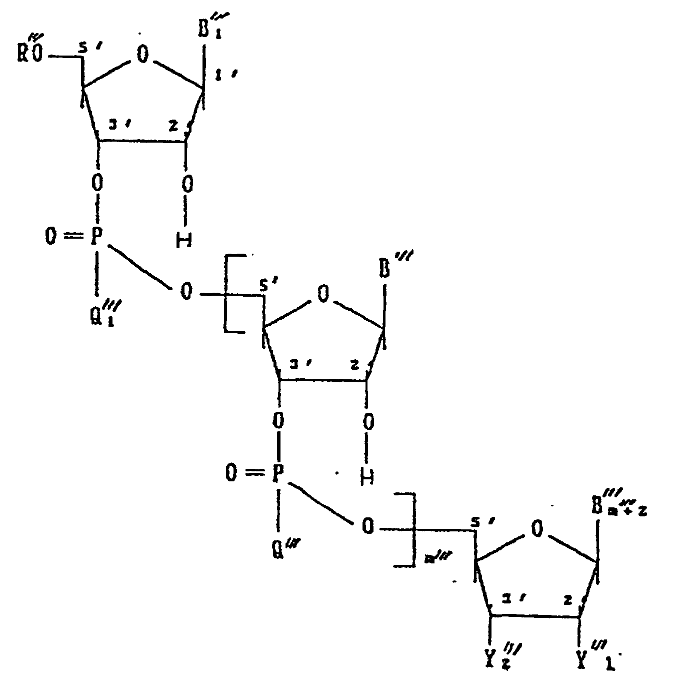

wherein:
m" ' is an integer of 4 to 50;
B" ' represents B" '_{2'} B" '_{3'} ····· B" '_{(m" '+1)'} and, B" '₁ through B" '_{(m" '+2)}, which may be the same or different, each represents any one of hypoxanthine-9-yl, guanine-9-yl, adenine-9-yl, cytosine-l-yl, uracil-1-yl and thymine-1-yl (provided that all of them do not represent adenine-9-yl);
Q" ' represents Q" '₂, Q" '₃, ..... Q" '_{(m" '+1)} and, Q" '₁ through Q" '_{(m" '+1)}, independently represents any one of a thio anion, an oxo anion, an aryloxy group, an alkylamino group, an arylamino group, an alkylthio group and an arylthio group (provided that at least one of Q" '₁ to Q" '_{(m" '+1)} represents a thio anion);
R" ' represents any one of a hydrogen atom, an acyl group having 1 to 20 carbon atoms and a phosphoryl group which may optionally have a substituent;
Y" '₁ and Y" '₂ independently represents any one of a methoxy group, an acyloxy group having 1 to 20 carbon atoms, an alkyloxy group which may optionally have a substituent, hydroxyl group, an alkylsilyl group, a hydrogen atom and a phosphoryl group which may optionally have a substituent.

Herein the alkyl represents a straight or branched chain hydrocarbon residue having 1 to 30 carbon atoms and the aryl represents a hydrocarbon residue having 6 to 30 carbon atoms including phenyl group. Examples of the substituent include cyanoethyl group, chlorophenyl group and a hydrocarbon residue having 1 to 20 carbon atoms.

With respect to the antiviral activity of oligodeoxynucleotide, an example of using oligodeoxynucleotide for inhibiting proliferation of Raus sarcoma virus without converting it into any derivative is reported (Zamecnik, P.C., Proc. Natl. Acad. Sci. USA, 75, 280-284, 1978). On the other hand, it is reported that oligodeoxynucleotide is rapidly decomposed in culture of mammal cells, cell extracts or sera (Wickstrom, E., Journal of Biochemical and Biophysical Methods, 13, 97-102, 1986). Accordingly, in order to expect a more effective antiviral activity of oligodeoxynucleotide, it is desired that the oligodeoxynucleotide be converted into such derivatives that are not decomposed. It is reported that for example, derivatives obtained by converting the phosphodiester in oligodeoxynucleotide into methylphophonate inhibit proliferation of herpes simplex virus type I (Smith, C.C., et al., Proc. Natl. Acad. Sci. USA, 83, 2787-2791, 1986). It is also reported that derivatives obtained by converting the phosphodiester in oligodeoxynucleotide into phosphorothioate have an improved stability (Stein, C.A., Nucleic Acids Res., 16, 3209-3221, 1988). It is further reported that these derivatives inhibit proliferation of AIDS virus (Matsukura, M., et al., Proc. Natl. Acad. Sci. USA, 84, 7706-7710, 1987). It is further reported that by leading to phosphorothioate derivatives in double stranded poly RNA or an interferon inducer, antiviral activity against bovine vesicular stomatitis virus is enhanced (De Clercq, E., et al., Virology, 42, 421-428, 1970).

On the other hand, it is known that 2'-O-methylribooligonucleotide takes a structure of the 2'-hydroxy group in the sugar moiety of RNA being methylated and has the property showing resistance to various enzymes (nucleases) for decomposing DNA or RNA (Gray, M.W., et al., Biochem. Biophys. Acta, 134, 243, 1967; Dunlap, B.E., et al., Biochemistry, 10, 2581-2587, 1971). Furthermore, 2'-O-methylribooligonucleotide has the property that it forms a stable duplex with RNA having its complementary sequence and the stability is significantly better than the stability of DNA-RNA complementary double strand having the same nucleotide sequence (Inoue, H., et al., Nucleic Acids Res., 15, 6131-6148, 1987). It is also known that 2'-O-methylribooligonucleotide is used for site-specific cleavage of RNA, utilizing the property resistant to decomposition from RNase H (Inoue, H., et al., FEBS Letters, 215, 327-330, 1987). In addition, 2'-O-methylribooligonucleotide is used to produce a unidirectional deletion mutant of DNA, utilizing the property showing significant resistance to DNase Bal3l nuclease as compared to DNA (Mukai, S., et al., Nucleic Acids Symposium Series, No. 19, 117-120, 1988). The foregoing findings are based on the characteristics that 2'-O-methylribooligonucleotide forms a stable duplex with RNA having its complementary nucleotide sequence and shows resistance various enzymes (nucleases) for decomposing DNA or RNA. Moreover, it was reported in the past that poly-2'-O-methylribooligonucleotide inhibited the progress of cancer in mice caused by retro virus, while its mechanism was clearly unknown (Tennant, R.W., et al., Proc. Natl. Acad. Sci., USA, 71, 3167-3171, 1974). In addition, 2'-O-methylribooligonucleotide can be prepared as in oligoribonucleotide using less expensive raw materials than in deoxyoligonucleotide.

With respect to the structure and properties of AIDS virus, the following findings have been made according to recent studies. That is, AIDS virus is retro virus having a single stranded RNA of about 9000 nucleotides in length as the substance of gene and has reverse transcriptase. Its nucleotide sequence (primary structure) of gene is already reported (Ratner, L., et al., Nature, 313, 277-284, 1985; Muesing, M.A., et al., Nature, 313, 450-458, 1985; Sanchez-Pescador, R., et al., Science, 227, 484-492, 1985; Wain-Hobson, S., et al., Cell, 40, 9-17, 1985). Target cells of infection are helper/inducer cells which are T4 (antigen detected with a monoclonal antibody) positive as surface antigen among human lymphocytes and after infection, cause these cytopathic effects and break down. The virus after adsorption to and invasion into the cells changes to linear double stranded viral DNA by reverse transcriptase possessed by themselves utilizing tRNA^{Lys} as a primer, transfers into the nucleus to take a circular structure and is integrated into host cell chromosomal DNA to become provirus. Transcription from the provirus is effected by cell-derived RNA polymerase II as in messenger RNA (hereafter simply referred to as "mRNA") of host cells, whereby the provirus is translated into viral protein. This procedure is regulated by at least two gene products, i.e., tat (transactivator) and rev (regulator of expression of virion proteins) encoded by the virus per se. tat works as a transcription activation factor for virus upon the region called TAR element (transacting responsive element) immediately after the initiation site of transcription (Rosen, C.A., et al., Cell, 41, 813-823, 1985). The transcribed viral RNA becomes in part genome RNA encapsulated into virion and is in part utilized as mRNA for expressing gene. Several species of mRNA are present; RNA in full length for expressing viral antigen and reverse transcriptase, mRNA for expressing envelope glycoprotein which is shortened by being spliced once, mRNA which is further shortened by being spliced at least twice in order to express genes such as tat, rev, etc., and the like.

As described above, the present invention has been successful for inhibiting infection with and proliferation of AIDS virus by further derivating the phosphate moiety, taking advantages of the oligoribonucleotide.

As described above, the compound of the present invention is in the form that the phosphate in the oligomer is derivated. As a result of various investigations, it has been found that the derivatives containing a thiophosphate bond are particularly effective and the thiophosphate bond is not necessary for all of the phosphodiester bonds in the oligomer molecule. It has also been found that the inhibitory effect can be exhibited in any of the case that these nucleotide sequences are complementary to those of genomic RNA for AIDS virus or DNA integrated into chromosome and in the case that the nucleotide sequences are not necessary complementary.

In the present invention, the oligomer having a complementary sequence should preferably take as the target a region that plays an important role on viral gene.

The derivative (Compound XXVII) recited in claim 3 is complementary to a nucleotide sequence present downstream from the primer binding site on viral genomic RNA and, it can be expected to inhibit transcription and replication of virus.

The derivative (Compound XXVIII) recited in claim 4 is complementary to a nucleotide sequence right upstream gag gene initiation codon.

The derivative (Compound XXIX) recited in claim 5 is complementary to a nucleotide sequence in gag gene and, it can be expected to inhibit translation of gag protein.

It is noted that all these derivatives show a potent anti-AIDS viral activity, although mechanism on the activity has not been experimentally proven in detail.

On the other hand, as is described in publications (cf., Matsukura, M., et al., Proc. Natl. Acad. Sci. USA, 84, 7706-7710, 1987; Agrawal, S., et al., Proc. Natl. Acad. Sci. USA, 85, 7709-7083, 1988) regarding anti-AIDS viral activity using oligodeoxynucleotide derivatives, it is reported that oligodeoxynucleotide derivatives not complementary to the nucleotide sequence of AIDS viral genomic RNA or DNA integrated into chromosome also possess anti-AIDS viral activity, like the complementary derivatives.

On the other hand, the compounds represented by general formulae (1) and (4) in the present invention which can be produced using less expensive raw materials than in oligodeoxynucleotide derivatives exhibit the anti-AIDS viral activity, although these compounds are not necessarily complementary to the nucleotide sequence of AIDS viral genomic RNA or DNA integrated into chromosome. It has been made clear that among the compounds represented by general formula (1), for example, the derivatives (Compound XXX) recited in claim 6, the derivatives (Compound XXXI) recited in claim 7, the derivatives (Compound XXXIII) recited in claim 8, the derivatives (Compound XXXIV) recited in claim 9, the derivatives (Compound XXXV) recited in claim 10 and the derivatives (Compound XXXVI) recited in claim 12 exhibit the anti-AIDS viral activity, although these compounds are not complementary to the nucleotide sequence of AIDS viral genomic-RNA or DNA integrated into chromosome. It has also been made clear that for example, among the compounds represented by general formula (4), the derivatives (Compound XXIII) recited in claims 15 exhibit the anti-AIDS viral activity, although these compounds are not complementary to the nucleotide sequence of AIDS viral genomic RNA or DNA integrated into chromosome.

On the other hand, the compounds in the present invention have low molecular weights unlike high molecular weight double stranded RNA known as a potent interferon inducer (cf., Lanpson, G.P., et al., Proc. Natl. Acad. Sci. USA., 58, 782, 1967; Field, A.K., et al., Proc. Natl. Acad. Sci. USA., 58, 1004, 1967) and derivatives thereof (De Clercq, E., et al., Virology, 42, 421-428, 1970). The compounds in the present invention are also clearly distinguished over 2'-5' oligoA all composed of adenosines and containing 2'-5' bond which is a protein synthesis inhibitor found in cells treated with interferon (cf., Kerr, I.M., Proc. Natl. Acad. Sci. USA., 75, 256, 1978) and derivatives thereof.

The oligoribonucleotide derivatives of the present invention can be produced by known methods such as the amidite method (cf., Matteucci, M.D., et al., Tetrahedron Lett., 22, 719, 1980), the H-phosphonate method (cf., Froehler, B.C. et al. Tetrahedron Lett., 29, 469, 1986; Garegg, P.J., Tetrahedron Lett., 27, 4055, 1986) and the like.

Furthermore, the protected 3'-O-methylnucleoside derivatives are produced according to known methods (cf., Robins, M.J., et al., J. Org. Chem., 39, 1891, 1974; Heikkila, J., et al., Acta Chem. Scand., B36, 715, 1982) can be led to the 3'-o-methylnucleoside-H-phosphonate derivatives by known methods (cf., Hata, T., et al., J. Am. Chem. Soc., 96, 7363, 1974; Sekine, M., et al., Tetrahedron Letters, 29, 1037-1040, 1988), which can be provided as raw materials for producing the oligoribonucleotide derivatives represented by general formulae (1) and (4).

In the oligoribonucleotide derivatives represented by general formulae (1) and (4), raw materials for producing the derivatives are, for example, protected 2'(or 3')-O-(tert-butyldimethylsilyl)ribonucleosides, which can easily be produced by a known method (Ogilvie, K.K., Can. J. Chem., 51, 3799, 1973); these derivatives can be led to the H-phosphonate derivatives in a manner similar to described above.

Using the protected novel 3'-O-methylribonucleotide derivatives and novel nucleoside derivatives produced by the foregoing processes, the oligoribonucleotide derivatives can be produced, for example, by known methods (cf., Froehler, B.C., et al., Tetrahedron Letters, 27, 469-472, 1986; Garegg, P.J., et al., Tetrahedron Letters, 27, 4051-4054, 1986). That is, the nucleotide derivative elements are sequentially condensed novel nucleoside derivatives as the polymer support, having acyl chloride to elongate the chain. Then, oxidation is performed using various oxidizing agents such as sulfur (S₈), iodine (I₂) or an alkylamine/carbon tetrachloride, etc. followed by removal of the protective group and purification.

Further in the compounds represented by general formulae (1) and (4) wherein the substituents shown by Q Q" ' are thio anions or thio anions and oxo anions, these compounds can also be produced by the amidite method (cf., Matteucci, M.D., et al., Tetrahedron Lett., 22, 719, 1980; Shibahara, S., et al., Nucleic Acids Res., 15, 4403, 1987; Usman, N., et al., J. Am. Chem. Soc., 109, 7845, 1987). In this case, the monomer may be oxidized with (S₈) or iodine (I₂) in the oxidation step after the condensation and then subjected to chain elongation followed by removal of the protective group and purification.

Further in the compounds which contain at the 5'-terminal thereof, for example, an alkyl-substituted thiophosphoryl group, an alkyl-substituted phosphoryl group or an alkyl-substituted alkylaminophosphoryl group, the chain elongation of the oligomer is effected on the polymer support by the process described above; after completion of the oxidation, the oxidation product is condensed with an alkyl-H-phosphonate and the condensation product is oxidized with sulfur (S₈), iodine (I₂) or an alkylamine/carbon tetrachloride followed by removal of the protective group and purification. The derivatives in which cholesterol is bound at the 5'-terminal via a spacer can be likewise obtained by condensing with mono(monomethoxytritylated) alkanediol H-phosphonate, treating with an acid, condensing with cholesteryl-H-phosphonate, oxidizing, removing the protective group and purification.

On the other hand, the compounds containing, for example, a hydroxyalkylphosphoryloxy group or a hydroxyalkylthiophosphoryloxy group at the 3'-terminal thereof can be obtained by condensing, oxidizing, removing the protective group and purification in a similar manner, using as a starting material a polymer support having bound thereto an alkanediol monosuccinate.

The anti-AIDS viral activity can be determined by the method discribed in a publication (cf., Harada, S., et al., Science, 229, 563-566, 1985), using HTLV-III_{B} (cf., Popovic, M., et al., Science, 224, 497-500, 1984) which is an isolated strain of AIDS virus. According to this method, MT-4 cells or HTLV-I positive cell line is used as the target cell so that this method is highly sensitive to viral infection and can exhibit the cytopathic effect highly efficiently. Using this method, the cytopathogen inhibitory effect was examined with Compound XXIII, after the compound was diluted with medium into various concentrations. As the result, the cytopathogen inhibitory effect and the effect of inhibiting development of viral specific antigen positive cells were noted with Compound XXIII substantially completely inhibited cytopathogen in a concentration of 3. 8 µM and appearance of viral specific antigen positive cells in a concentration of 7.5 µM, respectively. No cytotoxicity was detected with these compounds even in the maximum concentrations (60 µM).

As is clear from the foregoing, the novel oligoribonucleotide derivatives in accordance with the present invention have the anti-AIDS viral activity and are expected to use as anti-AIDS drugs.

The antiviral agent of the present invention may be used in a range of 0.01 to 2,000 mg, preferably 0.02 to 500 mg.

The effective component may be used in the form of, for example, a capsule, an elixir, a microcapsule or a suspension.

The compound used as the effective component of the present invention can be administered to the patient who requires treatment or prophylaxis, in a dose of 0.01 to 1,000 mg per person, generally several times in a daily dose of 0.02 to 2,000 mg. The dose can be varied depending upon condition of disease, body weight of the patient and other factors recognized by one skilled in the art.

The compound used in the present invention or physiologically acceptable salt compound or a mixture thereof can be mixed in an amount of approximately 0.2 to 500 mg together with vehicles, carriers, diluents, binders, antiseptics, stabilizers, flavors, etc. in a unit dose form required to make pharmaceutical preparations generally admitted. An amount of the active substance in these compositions or preparations is incorporated to give an appropriate dose in a range indicated.

Specific examples of drugs which can be incorporated into tablets, capsules, etc. are given below: binders such as tragacanth, gum arabic, corn starch or gelatin; excipients such as fine crystalline cellulose; swelling agents such as corn starch, pregelatinized starch, alginic acid, etc.; lubricants such as magnesium stearate; sweeteners such as sucrose, lactose or saccharine; flavors such as peppermint, Gaultheria adenothrix oil or cherry, etc. In case that the preparation unit form is a capsule, a liquid carrier such as oils and fats can additionally be incorporated into the materials of type described above. A variety of other materials may be incorporated as protectives or in order to change physical form of the preparation unit by another method. For example, tablets can be coated with shellac or sugar or with both of them. Syrup or elixir may contain sucrose as a sweetener, methyl- and propylparabens as antiseptics and pigments and flavors such as cherry or orange flavor.

In the case of enteric coating, for example, 8% aqueous solution of hydroxyphenylmethyl cellulose is used as a coating pretreatment agent, 10% aqueous solution of hydroxypropylmethyl cellulose phthalate and 3% aqueous solution of polyacetyne are used as coating agents. They are used respectively to make enteric coating preparations in a conventional manner.

A sterile composition for injection can be formulated in a conventional manner to prepare pharmaceutical preparations by dissolving or suspending the active substance, naturally occurring plant oils such as sesame oil, coconut oil, peanut oil, cotton seed oil, etc. or synthetic fatty vehicle such as ethyl oleate in a vehicle such as water for injection. Buffer agents, antiseptics, antioxidants, etc. may be added, if necessary.

### Examples

Hereafter the present invention is concretely described by referring to the examples below.

### Example 1. Production of 5'-O-monomethoxytrityl-N²-isobuty|yl-3'-O-methylguanosine-bound controlled pore glass:

5'-O-monomethoxytrityl-N²-isobuty|yl-3'-O-methylguanosine synthesized in a conventional manner was led to 2'-O-succinylpentachlorophenyl ester by a known process (cf., Miyoshi, K., et al., Nucleic Acids Res., 8, 5491, 1980). That is, 639 mg (1 mmol) of 5'-O-monomethoxytrityl-N²-isobuty|yl-3'-O-methylguanosine and 183 mg of 4-N,N-dimethylaminopyridine (merely preferred to as DMAP) were dissolved in 4 ml of absolute pyridine. While stirring at room temperature, 150 mg (1.5 mmol) of succinic anhydride was added to the solution. After stirring for 90 minutes, the reaction liquid was distilled under reduced pressure. The resulting oily residue was dissolved in 35 ml of chloroform. The solution was washed 3 times with 30 ml of 0.1 M triethylamine-bicarbonate buffer (pH 7.5). After drying over anhydrous sodium sulfate, the chloroform layer was evaporated to dryness under reduced pressure. The obtained residue was dissolved in 5 ml of dimethylformamide (DMF) and 400 mg (1.5 mmol) of pentachlorophenol and 309 mg (1.5 mmol) of dicyclohexylcarbodiimide were added to the solution followed by stirring at room temperature for 13.5 hours. The precipitated insoluble matters were removed by filtration and the filtrate was removed by distillation under reduced pressure. The resulting residue was dissolved in 3 ml of chloroform and the solution was purified by column chromatography using a column packed with 20 g of silica gel 60. That is, elution was carried out using as the mobile phase, in sequence, 100 ml of chloroform, 100 ml of chloroform/0.3% methanol, 100 ml of chloroform/0.5% methanol and further 100 ml of chloroform/0.6% methanol and the resulting pure fractions of 5'-O-monomethoxytrityl-N²-isobuty|yl-3'-O-methylguanosine-2'-O-succinyl-pentachlorophenyl ester were collected and evaporated to dryness under reduced pressure to give 530 mg of the ester in yield of 53.6% (Rf value: 0.73 when developed with chloroform : methanol = 20 : 1 in silica gel 60 thin layer chromatography). Next, the whole amount (530 mg) of the ester was dissolved in 5.4 ml of DMF; on the other hand, 1 g of controlled pore glass (CPG/long chain alkylamine, pore diameter: 500 Å, particle size: 122-177 µ, NH₂-: 30 µmols/g, manufactured by Pierce Chemical Inc.) was charged in a reactor equipped with a glass filter and washed with 1 ml of DMF followed by drying for a minute in an argon gas flow. The aforesaid pentachlorophenyl ester DMF solution, 5.4 ml, and 73 µl of triethylamine were added to the pore glass, respectively. After sealing, the reaction was carried out at 30°C overnight. The reaction liquid was filtered under an argon pressure. After washing 3 times with 5 ml of DMF and then 3 times with 5 ml of tetrahydrofuran (THF), the controlled pore glass was dried for a minute in an argon flow. Next, a liquid mixture of 410 mg of DMAP, 1.4 g of 2,6-lutidine, 660 µl of acetic anhydride and 6 ml of THF was added to the pore glass. The mixture was shaken at 30°C for 15 minutes to acetylate the unreacted amino group. After filtration, the reaction mixture was washed 3 times with 5 ml of THF and then 3 times with 5 ml of diethyl ether and dried in an argon flow to give 1 g of 5'-O-monomethoxytrityl-N²-isobutylyl-3'-O-methylguanosine-bound controlled pore glass. A small amount of the product was weighed and the monomethoxytrityl group was cleaved with 3% trichloroacetic acid. The isolated tritanol was subjected to quantitative colorimetry with a perchloric acid-ethanol mixture. As the result, the bound amount was 26 µmols/g.

Furthermore, 5'-O-dimethoxytrityl-N⁶-benzoyl-3'-O-methyladenosine-bound controlled pore glass, 5'-O-dimethoxytrityl-3'-O-methylinosine-bound controlled pore glass, 5'-O-dimethoxytrityl-3'-O-methyluridine-bound controlled pore glass, 5'-O-dimethoxytrityl-N⁴-benzoyl-3'-O-methylcytidine-bound controlled pore glass, 5'-O-dimethoxytrityl-2'-O-(tert-butyldimethylsilyl)inosine-bound controlled pore glass and monomethoxy tritylhexanediol-bound controlled pore glass were produced, respectively, in a manner similar to the above.

### Example 2 Production of Compounds XXVII, XVIII and XXIX

N²-Isobuty|yl-5'-O-monomethoxytrityl-3'-O-methylguanosinebound polymer support, 40 mg (binding amount: 1.0 µmol), was packed in a cartridge and the cartridge was set in a DNA synthesizer (model 380A, manufactured by Applied Biosystems Inc.). Following the procedure shown in Table 1, each of compounds XXVII, XXVIII and XX were treated using the following compounds : N⁴ benzoyl-5'-O-dimethoxytrityl-2'-O methylcytidine-3'-O-(H-phosphonate) [Compound I], N⁶-benzoyl-5'-O-dimethoxytrityl-2'-O-methyladenosine-3'-O-(H-phosphonate) [Compound II], N²-isobutylyl-5'-O-monomethoxytrityl-2'-O methylguanosine-3'-O-(H-phosphonate) [Compound III], and 5'-O-dimethoxytrityl-2'-O-methyluridine-3'-O-(H-phosphonate). After repeating 19 cycles, the intermediate for preparing the polymer support was withdrawn from the cartridge and shifted to a reactor equipped with a glass filter. After washing with 1 ml of acetonitrile, the intermediate was dried in an argon gas flow. Next, 2 ml of a solution of 0.2 M sulfur (S₈) in triethylamine : carbon disulfide : pyridine (1 : 12 : 12, volume ratio) was added to the intermediate. After shaking, the mixture was allowed to stand for 12 hours. The reaction mixture was filtered and the intermediate for synthesizing the polymer support was washed 5 times with 2 ml of carbon disulfide and once with 2 ml of diethyl ether. After drying in an argon flow, the intermediate was transferred to a glass-made bial of a 3 ml volume. 28% Ammonia water, 2 ml, was charged in the bial and sealed followed by heating at 55°C for 5 hours. The polymer support was removed by filtration and the reaction liquid was evaporated to dryness under reduced pressure. The resulting residue was dissolved in 300 µl of 0.1 M triethyl ammonium-acetic acid buffer (pH 7.0, hereafter simply referred to as TEAA) containing 10% acetonitrile. The solution was purified through a column having a diameter of 1 cm and a length of 12 cm, packed with reverse phase silica gel (Waters Co., Ltd., Prep PAK-500/C-18). That is, 0.1 M TEAA buffer (pH 7.0) having a linear gradient of 10% to 35% acetonitrile was used as the mobile phase and quantitative determination was performed at absorbancy at a wavelength of 254 nm. After the solvent was distilled off under reduced pressure, the residue was co-evaporated together with 2 ml of water 3 times and 3 ml of 80% acetic acid was added thereto followed by stirring for 10 minutes. After the reaction liquid was evaporated to dryness, the residue was further co-evaporated together with water to remove acetic acid. To the residue was added 1.5 ml of 0.1 M triethylamine-bicarbonate buffer (pH 7.5) containing 10% acetonitrile. The mixture was washed twice with 1.5 ml of diethyl ether. The aqueous layer was evaporated to dryness and 200 µl of 0.1 M TEAA buffer (pH 7.0) containing 10% acetonitrile was added to the resulting residue. After passing through a 0.45 µ filter (manufactured by Millipore Corp.), purification was performed by high performance liquid chromatography. That is, YMC Pack ODS (manufactured by Yamamura Science Co., Ltd.) column was used and 0.1 M TEAA buffer (pH 7.0) having a linear gradient. of 10% to 70% acetonitrile was used as the mobile phase. The main peak eluted at a flow rate of 1.2 ml/min was fractionated to yield purified compounds XXVII, XXVIII and XXIX, respectively.

Each compound was dissolved in D₂O containing 10 mM triethylamine-bicarbonate buffer (pH 7.5) and ³¹P-NMR was measaured (device for measurement: JEOL JMS-DX300, manufactured by JEOL Inc.).

**Table 1**

| One Cycle of Procedure for Chain Elongation Reaction | | | |
|---|---|---|---|
| Operation Number | Name of Reagent or Operation | Liquid Feeding Time (second) | Number of Time |
| 1. | Washing with acetonitrile | 30 | 1 |
| 2. | Drying in argon | 20 0 | 1 |
| 3. | 3% Trichloroacetic acid/ dichloromethane | 30 | 6 |
| 4. | Drying in argon | 5 | 1 |
| 5 | Washing with acetonitrile | 30+60 | 2 |
| | Drying in argon | 5+20 | |
| 6. | 0.2 M Compounds I-V, XV-XIII, XXI or XXII/acetonitrile 1·0 M Pivaloyl chloride/ pyridine | 13 | 1 |
| 7. | Allowing to stand | 180 | 1 |
| 8. | Drying in argon | 15 | 1 |
| 9. | Washing with acetonitrile | 30 | 3 |
| | Drying in argon | 20 | |

### Example 3 Production of Compound XXIII

5'-O-Dimethoxytrityl-2'-O-(tert-butyldimethylsilyl)inosine-CPG (controlled pore glass), 40 mg (binding amount: 1.0 µmol) each, was packed in 3 cartridges and the cartridges were set in a DNA synthesizer (model 380A, manufactured by Applied Biosystems Inc.). Following the procedure shown in Table 1, 5'-O-dimethoxytrityl-2'-O-(tert-butyldimethylsilyl)inosine-3'-O-(H-phosphonate) (Compound XXI) was used together with pivaloyl chloride. After repeating 19 cycles, the intermediate for preparing the polymer support was withdrawn from each cartridge and shifted to a reactor equipped with a glass filter. After washing with 1 ml of acetonitrile, the intermediate was dried in an argon gas flow. Next, 2 ml of a solution of 0.2 M sulfur (S₈) in triethylamine : carbon disulfide : pyridine (1 : 12 : 12, volume ratio) was added to the intermediate. After shaking, the mixture was allowed to stand for 2 hours. The reaction liquid was filtered and the intermediate for synthesizing the polymer support was washed 5 times with 2 ml of carbon disulfide and once with 2 ml of diethyl ether. After drying in an argon flow, the intermediate was transferred to a glass-made bial of a 3 ml volume. 28% Ammonia water, 1.5 ml, and 0.5 ml of ethanol were charged in the bial and sealed followed by heating at 55°C for 5 hours. The polymer support was removed by filtration and washed with 1 ml of ethanol and then with 1 ml of 50% ethanolic water. The filtrates were combined and evaporated to dryness. To the resulting residue was added 1.5 ml of THF solution (1 M) of tetra-(n-butyl)ammonium fluoride. The mixture was shaken at room temperature for 17 hours. Next, 1 ml of 20 mM triethyl ammonium-bicarbonate buffer (pH 7.0, hereafter simply referred to as TEAB) to dilute. The dilution was purified by gel filtration chromatography using a Sephadex G25-packed column (diameter of 1.6 cm, length of 5 cm, NAP-25, manufactured by Pharmacia Inc.). That is, after the reaction dilution described above was added, elution was performed using 20 mM TEAB buffer as the mobile phase and passed-through fractions were collected and evaporated to dryness. The residue was dissolved in 300 µl of 0.1 M triethylamine-acetate buffer (pH 7.0, hereafter simply referred to as TEAA) containing 10% acetonitrile and the solution was purified through a column packed with reverse phase silica gel (Waters Co., Ltd., Prep PAK-500/C-18). That is, 0.1 M TEAA buffer having a linear gradient of 10% to 40% acetonitrile was used as the mobile phase and quantitative determination was performed at absorbancy at a wavelength of 254 nm to give the fraction of Compound XXIII having a dimethoxytrityl group at the 5'-terminal. After the solvent was distilled off, the residue was co-evaporated together with water 3 times and 3 ml of 80% acetic acid was added thereto followed by stirring for 10 minutes. After the reaction liquid was evaporated to dryness, the residue was further co-evaporated under reduced pressure together with water to remove acetic acid. To the residue was added 1.5 ml of 20 mM TEAB buffer. The mixture was washed twice with 1.5 ml of ethyl acetate. The aqueous layer was evaporated to dryness and 200 µl of 0.1 M TEAA buffer containing 10% acetonitrile was added to the resulting residue. The mixture was purified by high performance liquid chromatography. That is, YMC Pack ODS (manufactured by Yamamura Science Co., Ltd.; diameter of 6 mm, length of 30 cm) column was used and 0.1 M TEAA having a linear gradient of 10% to 50% acetonitrile was used as the mobile phase. The main peak eluted at a flow rate of 1.2 ml/min was fractionated to give 126
OD^{260 nm} units (about 514 nmols) of Compound XXIII in yield of 12.9%.

In a manner similar to the procedure described above, Compound XXIV was obtained in 65 OD^{260 nm} units (about 264 nmols) showing the overall yield of about 11%, using as the starting material 2.4 µmols of 5'-O-dimethoxytrityl-2'-O-(tert-butyldimethylsilyl)inosine-bound CPG and repeating 19 cycles in the condensation cycle using 5'-O-dimethoxytrityl-3'-O-(tert-butyldimethylsilyl)inosine-2'-O-(H-phosphonate) (Compound XXII) together with pivaloyl chloride.

Compound XXIII was dissolved in D₂O containing 10 mM TEAB buffer (pH 7.5) and ³¹P-NMR was measaured (device for measurement: JEOL GX-400FT-NMR manufactured by JEOL Inc.). When trimethyl phosphate was used as the external standarad, Compound XXIII showed the characteristic signal alone at 53-54 ppm.

Furthermore, Compounds XXXIII, XXXIV and XXXV were produced in a manner similar to the procedure described above.

On the other hand, Compound XXX was produced by, in the production steps, performing the acid treatment after completion of the final condensation cycle, condensing with monomethoxytrityl-hexanediol-H-phosphonate, then performing the acid treatment, finally condensing with cholesteryl-H-phosphonate and thereafter carrying out in a similar manner. Compound XXXI was also produced by finally condensing with stearyl-H-phosphonate and thereafter carrying out in a similar manner.

Compound XXXVI was produced using monomethoxytrityl-hexanediolbound controlled pore glass as the starting material, by treating with an acid after completion of the condensation cycle, then finally condensing with monomethoxytrityl-hexanediol-H-phosphonate and thereafter carrying out in a manner similar to the production previously described.

### Example 4 Test on anti-AIDS virus activity

Compound XXIII was dissolved in 10% fetal calf serum-containing RPMI 1640 medium in a definite concentration. MT-4 cells infected with AIDS virus (HTLV-III_{B} strain, 3 x 10⁵ PFU/ml) at a MOI of 0.002 were mixed with the aforesaid medium containing oligonucleotide in 3 x 10⁵/ml at the initial stage followed by culturing at 37°C. After the cell state was observed on Day 3, the system was again ajusted to cell counts of 3 x 10⁵ to 5 x 10⁵/ml. Oligonucleotide was also freshly supplemented to have the same concentration as in the initial stage. Cytopathic effect with viral infection was evaluated 6 days after the infection. The results are shown in Table 2, indicating that symbol + denotes the case in which viral infection and proliferation are inhibited and the cells grow normally and symbol - denotes the case in that the cells are destructed due to viral infection. In any case, no cytotoxicity was noted in tested concentrations.

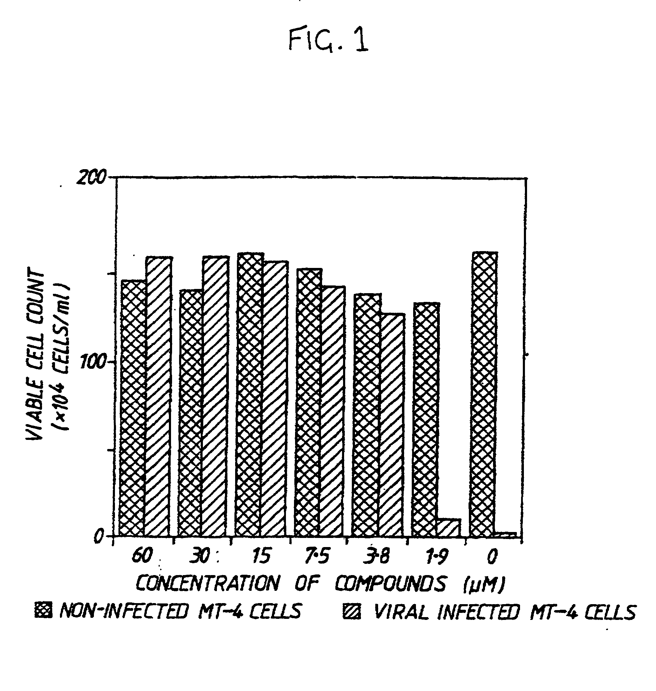

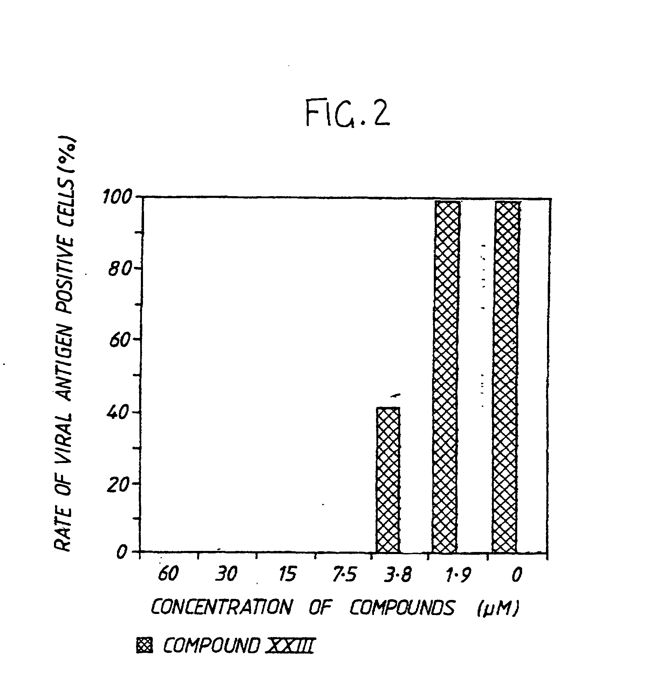

Regarding the results of Compound XXIII, the viable cell count on Day 6 after infection is shown in Table 2 and the rate of viral antigen positive cells on Day 6 after infection is shown in Table 3. The viable cell counts of Compound XXIII are shown by graph in Fig. 1. Furthermore, the rates of viral antigen positive cells of Compound XXIII on Day 6 after infection are shown by graphs in Fig. 2.

**Table 2**

| Anti-AIDS Viral Activity of Compound XXIII (viable cell count on Day 6 after infection: x 10⁴ cells/ml) | | | | | | | |
|---|---|---|---|---|---|---|---|
| Concentration of Compound (µM) | 60 | 30 | 15 | 7.5 | 3.8 | 1.9 | 0 |
| Compound XXIII: | | | | | | | |
| Non-infected | 147 | 141 | 161 | 152 | 138 | 133 | 161 |
| Infected | 159 | 159 | 156 | 142 | 127 | 10 | 2 |

**Table 3**

| Anti-AIDS Viral Activity of Compound XXIII (rate of viral antigen positive cells on Day 6 after infection: %) | | | | | | | |
|---|---|---|---|---|---|---|---|
| Concentration of Compound (µM) | 60 | 30 | 15 | 7.5 | 3.8 | 1 .9 | 0 |
| Compound XXIII | 0.2 | 0.2 | 0.2 | 0.2 | 41 | >90 | >90 |

As described above, the novel ribooligonucleotide phosphorothioate derivatives according to the present invention have anti-AIDS viral activity and are expected to be used as drugs such as drugs for treatment of AIDS. Therefore, the present invention is extremely useful in the drug industries.

Reference is made above to the accompanying drawings in which:
Fig. 1 shows the measurement results in Example 7 on viable cell count of Compound XXIII on Day 6 after infection; and
Fig. 2 shows the rate of viral antigen positive cells of Compound XXIII on Day 6 after infection, of Example 4.

## Claims

1. An oligoribonucleotide derivative represented by the general formula: 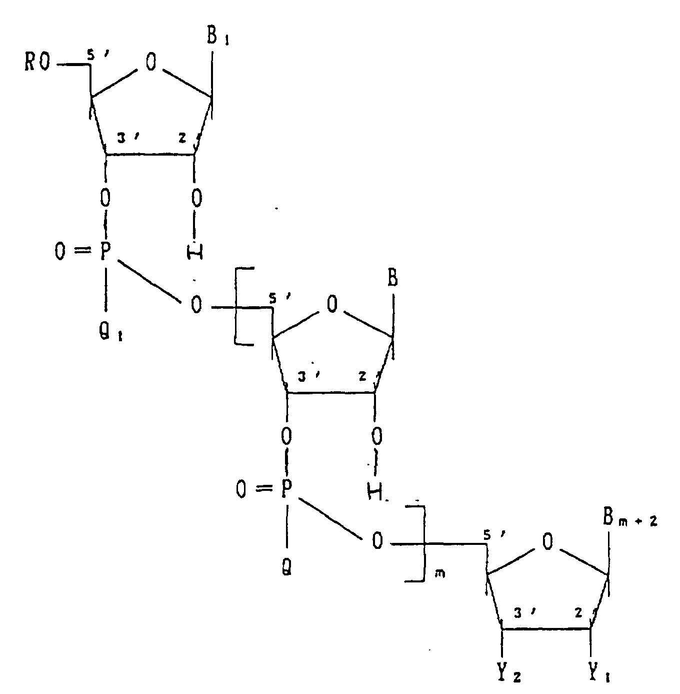 wherein:
m is an integer of 4 to 50;
B represents B₂, B₃, ..... B₍ₘ₊₁₎, and B₁ to B₍ₘ₊₂₎, which may be the same or different, each represents any one of hypoxanthine-9-yl, guanine-9-yl, adenine-9-yl, cytosine-1-yl, uracil-1-yl and thymine-1-yl;
Q represents Q₂, Q₃, ...... Q₍ₘ₊₁₎, and Q₁ to Q₍ₘ₊₁₎, independently represents any one of a thioanion, an oxoanion, an alkyl group, an aryloxy group, an alkylamino group, an arylamino group, an alkylthio group and an arylthio group (provided that at least one of Q₁ to Q₍ₘ₊₁₎ represents a thioanion) ;
R represents a hydrogen atom, 'an acyl group having 1 to 20 carbon atoms, a phosphoryl group which may optionally have a substituent, a thiophosphoryl group which may optionally have a substituent or an alkylaminophosphoryl group which may optionally have a substituent;
Y₁ and Y₂ independently represent a methoxy group, an acyloxy group having 1 to 20 carbon atoms, a hydrogen atom, a hydroxyl group, an alkyloxy group which may optionally have a substituent, a phosphoryloxy group which may optionally have a substituent, a thiophosphoryloxy group which may optionally have a substituent and an alkylaminophosphoryloxy group which may optionally have a substituent.

2. A derivative as claimed in claim 1, wherein said substituent on R, Y₁ and Y₂, which may be the same or different, each represents any one of a cyanoethyl group, a chlorophenyl group, a monophosphoryl group, a pyrophosphoryl group, a hydrocarbon residue having 1 to 20 carbon atoms, a cholesteryl group and a group having the formula J-(K-O-L)_{E} wherein J represents: 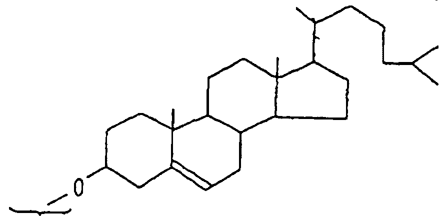 a hydroxyl group or any one of the oligoribonucleotidyl groups defined for the structural formula of said derivative according to claim 1, from which any one of RO, Y₁ and Y₂ is removed;
K represents any one of a phosphoryl group, a thiophosphoryl group and an alkylaminophosphoryl group having 1 to 10 carbon atoms;
L represents a hydrocarbon residue having 1 to 20 carbon atoms; and
E represents an integer of 1 to 10.

3. A derivative as claimed in claim 1, wherein m represents 25, B₁ to B₂₇ represents, in sequence, U, U, C, C, C, U, U, U, C, G, C, U, U, U, C, A, A, G, U, C, C, C, U, G, U, U, C and G; Q₁ to Q₂₆ each represent a thioanion; Y₁ and Y₂ represent OCH₃; and R represents H (compound XXVII).

4. A derivative as claimed in claim 1, wherein m represents 28, B₁ to B₃₀ represent, in sequence U, C, C, U, U, C, U, A, G, C, U, C, C, G, C, U, A, G, U, C, A, A, A, A, U, U, U and U; Q₁ to Q₂₉ each represent a thioanion; Y₁ and Y₂ represent OCH₃; and R represents H (compound XXIII).

5. A derivative as claimed in claim 1, wherein m represents 26, B₁ to B₂₈ represent, in sequence, U, U, U, U, A, A, U, U, U, A, U, A, U, U, U, U, U, U, C, U, U, U, C, C, C, C, C and U; Q₁ to Q₂₇ each represent a thioanion; Y₁ and Y₂ represent OCH₃; and R represents H (compound XXIX).

6. A derivative as claimed in claim 1, having the structural formula:

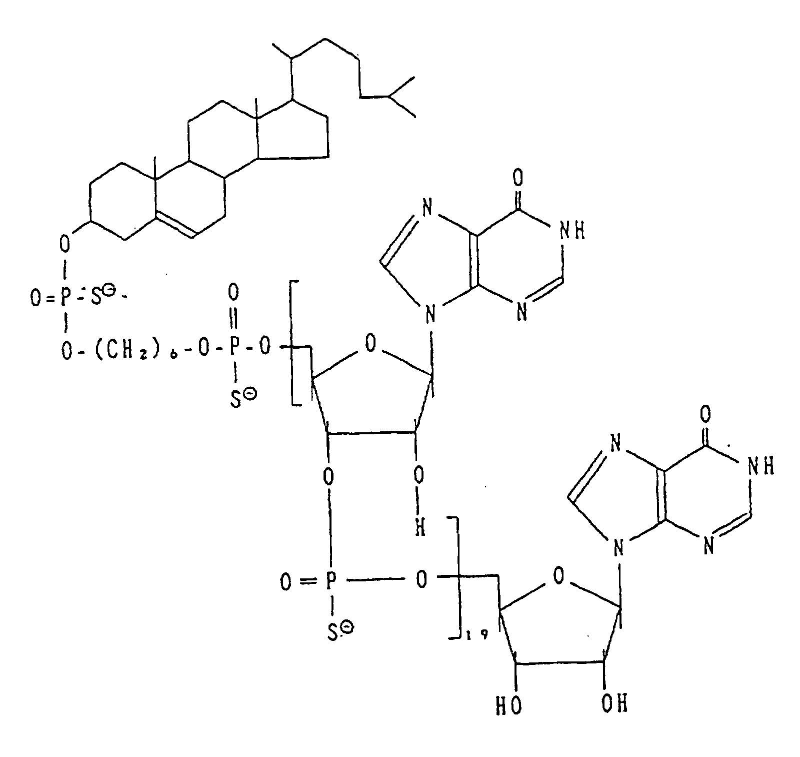

7. A derivative as claimed in claim 1, having the structural formula:

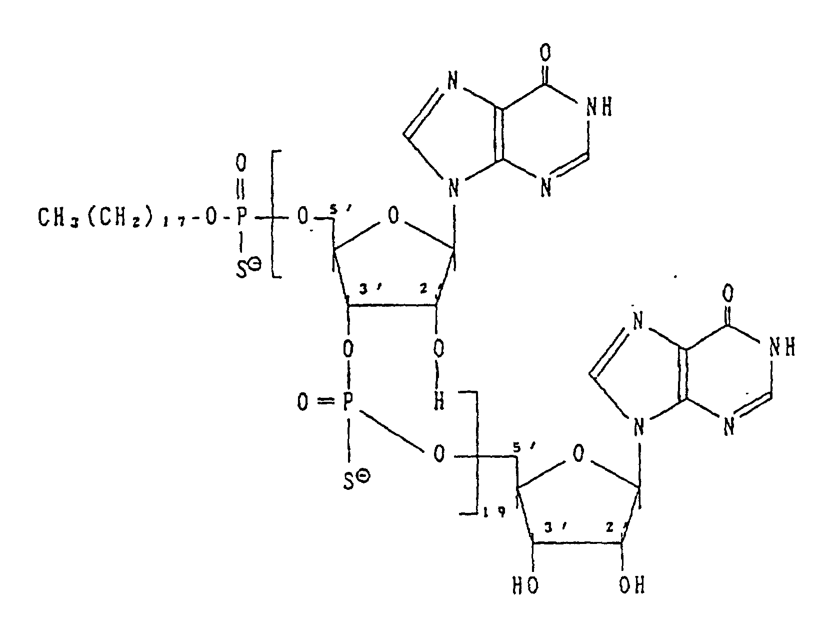

8. A derivative as claimed in claim 1, having the structural formula:

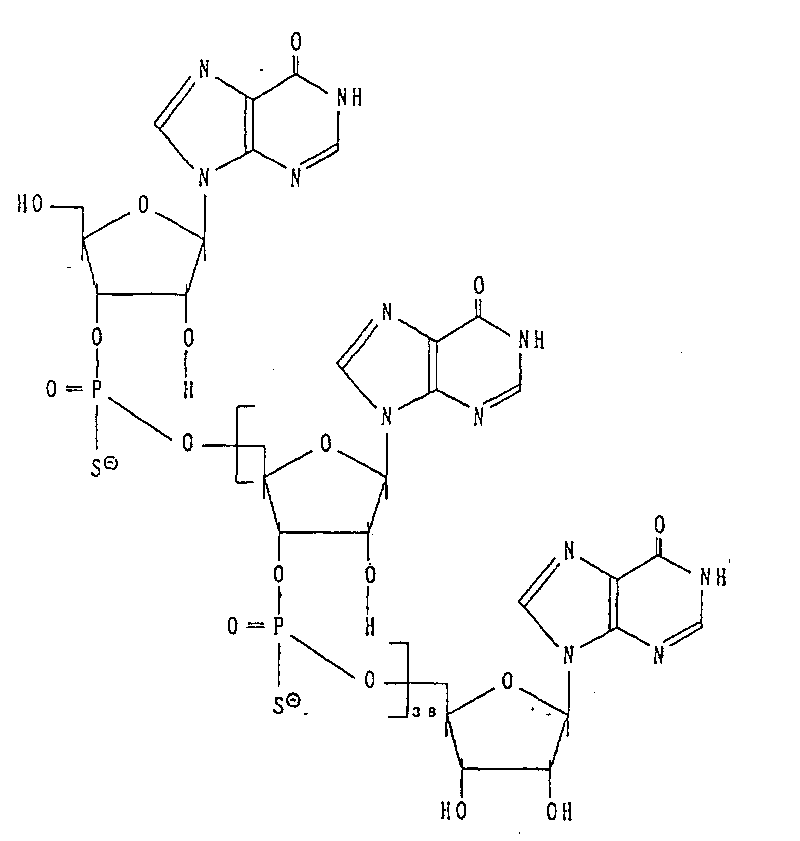

9. A derivative as claimed in claim 1, having the structural formula:

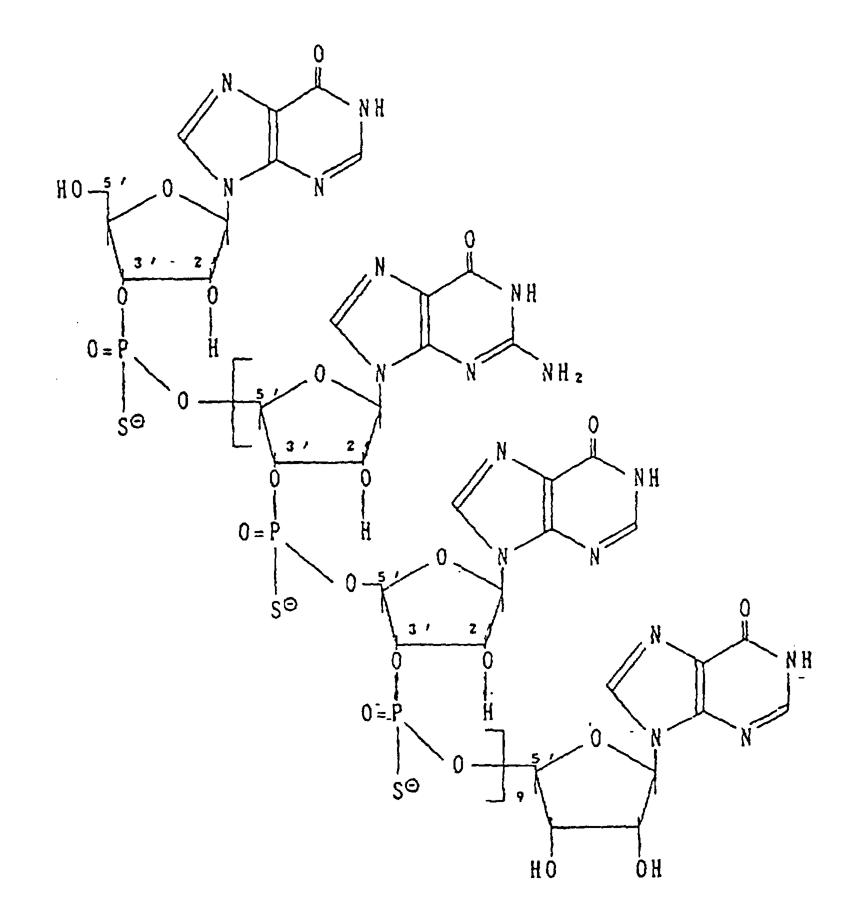

10. A derivative as claimed in claim 1, having the structural formula:

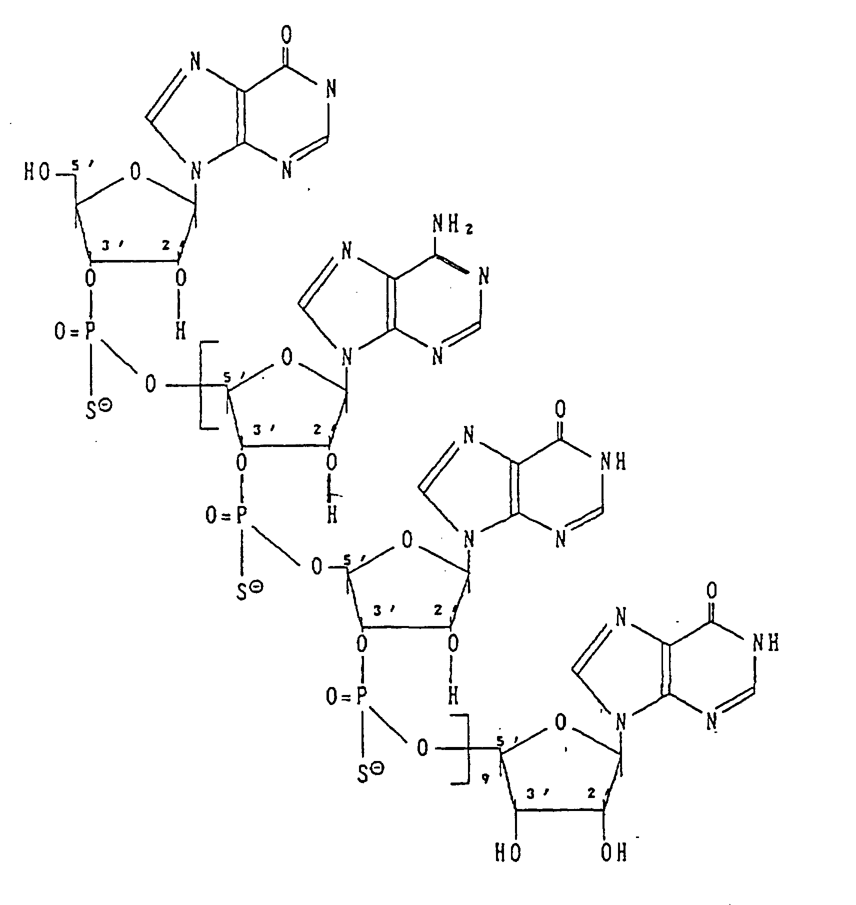

11. A derivative as claimed in claim 1, having the structural formula:

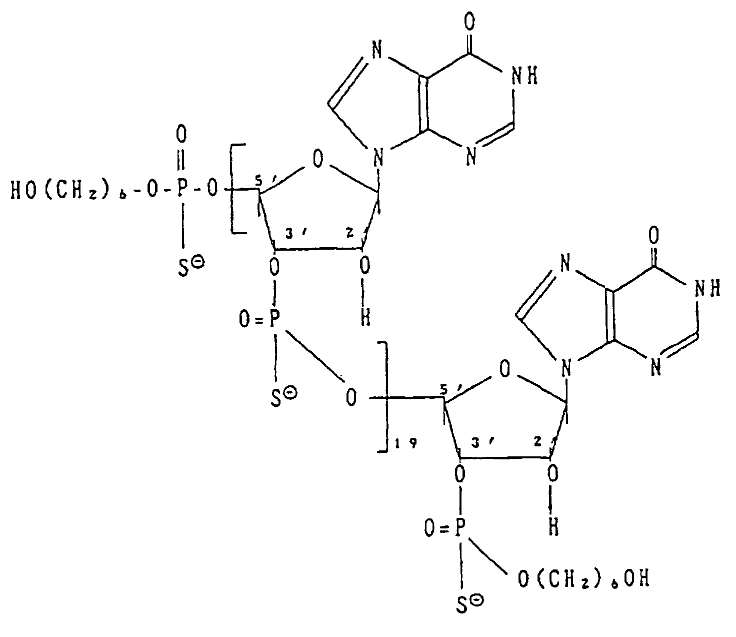

12. An anti-AIDS viral agent comprising as an effective ingredient at least oligoribonucleolide derivative as claimed in any of claims 1 to 11.

13. An oligoribonucleotide derivative represented by the general formula: 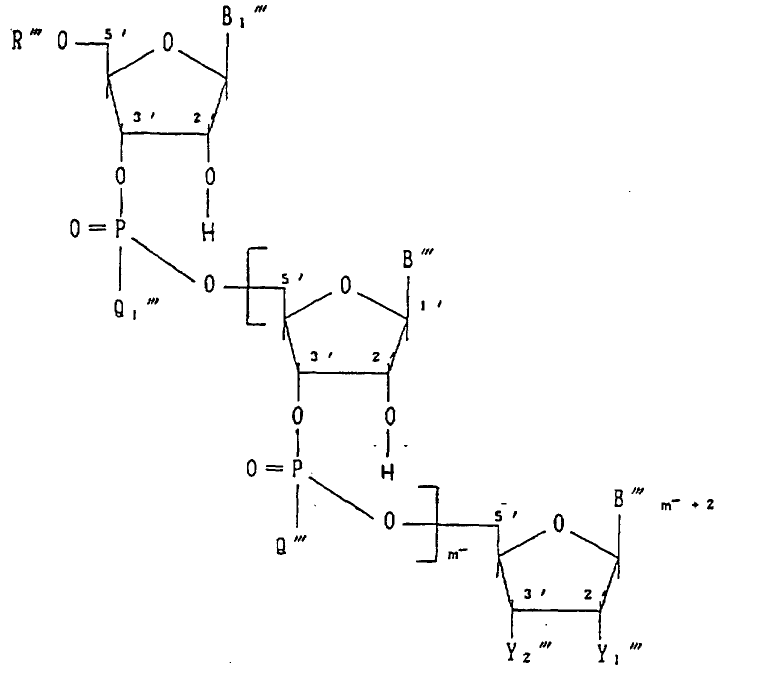 wherein:
m" ' is an integer of 4 to 50;
B" ' represents B" '₂, B" '₃, ..... B" '_{(m" '+1)}, and B" '₁ to B" '_{(m" '+2)} which may be the same or different, each represents any one of hypoxanthine-9-yl, guanine-9-yl, adenine-9-yl, cytosine-1-yl, uracil-1-yl and thymine-1-yl (provided that all of them do not represent adenine-9-yl) ;
Q" ' represents Q" '₂, Q" '₃, ..... Q" '_{(m" '+1)}, and Q" '₁ to Q" '_{(m" '+1)}, independently represents any one of a thioanion, an oxoanion, an aryloxy group, an alkylamino group, an arylamino group, an alkylthio group and an arylthio group (provided that at least, one of Q" '₁ to Q" '_{(m" '+1)}, represents a thioanion) ;
R" ' represents any one of a hydrogen atom, an acyl group having 1 to 20 carbon atoms and a phosphoryl group which may optionally have a substituent;
Y" '₁ and Y" '₂ independently represents any one of a methoxy group, an acyloxy group having 1 to 20 carbon atoms, an alkyloxy group which may optionally have a substituent, a hydroxyl group, an alkylsilyl group, a hydrogen atom and a phosphoryl group which may optionally have a substituent.

14. A derivative as claimed in claim 13, wherein said substituent is any one of a cyanoethyl group, a chlorophenyl group and a hydrocarbon residue having 1 to 20 carbon atoms.

15. A derivative as claimed in claim 13, having structural formula:

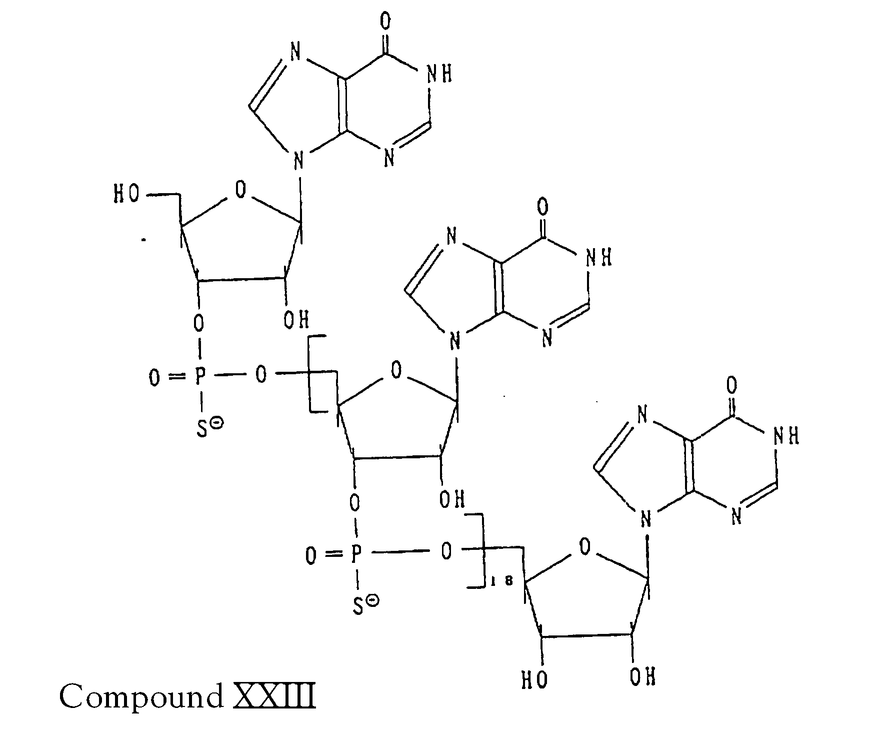

## Patentansprüche

1. Oligoribonukleotid-Derivat gemäß der allgemeinen Formel 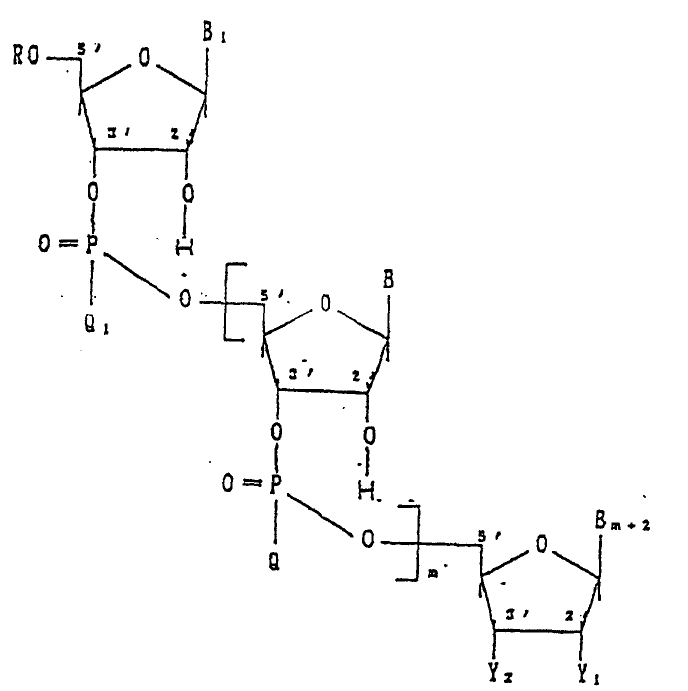 worin ist
m eine ganze Zahl von 4 bis 50;
B gleich B₂, B₃,...B₍ₘ₊₁₎, und B₁ bis B₍ₘ₊₂₎, welche gleich oder ungleich sein können und jeweils stehen für eine Gruppe aus Hypoxanthin-9-yl, Guanin-9-yl, Adenin-9-yl, Cytosin-1-yl, Uracil-1-yl und Thymin-1-yl;
Q gleich Q₂, Q₃...Q₍ₘ₊₁₎, und Q₁ bis Q₍ₘ₊₁₎ unabhängig jeweils ein Thioanion, ein Oxoanion, eine Alkyl-Gruppe, eine Aryloxy-Gruppe, eine Alkylamino-Gruppe, eine Arylamino-Gruppe, eine Alkylthio-Gruppe und eine Arylthio-Gruppe (vorausgesetzt, dass mindestens ein Rest aus Q₁ bis Q₍ₘ₊₁₎ ein Thioanion ist) ;
R gleich ein Wasserstoffatom, eine Acyl-Gruppe mit 1 bis 20 Kohlenstoffatomen, eine Phosphoryl-Gruppe, die wahlfrei einen Subtituenten haben kann, eine Thiophosphoryl-Gruppe, die wahlfrei einen Substituenten haben kann, oder eine Alkylaminophosphoryl-Gruppe, die wahlfrei einen Substituenten tragen kann;
Y₁ und Y₂ unabhängig eine Methoxy-Gruppe, eine Acyloxy-Gruppe mit 1 bis 20 Kohlenstoffatomen, ein Wasserstoffatom, eine Hydroxyl-Gruppe, eine Alkyloxy-Gruppe, die wahlfrei einen Substituenten haben kann, eine Phosphoryloxy-Gruppe, die wahlfrei einen Substituenten haben kann, eine Thiophosphoryloxyl-Gruppe, die wahlfrei einen Substituenten haben kann und eine Alkylaminophosphoryloxy-Gruppe, die wahlfrei einen Substituenten haben kann.

2. Derivat nach Anspruch 1, wobei der Substituent an R, Y₁ und Y₂, die gleich oder ungleich sein können, irgendeine Gruppe ist aus Cyanoethyl-Gruppe, Chlorophenyl-Gruppe, Monophosphoryl-Gruppe, Pyrophosphoryl-Gruppe, Kohlenwasserstoffrest mit 1 bis 20 Kohlenstoffatomen, Cholesteryl-Gruppe sowie eine Gruppe der Formel J-(K-O-L)_{E}, worin J steht für 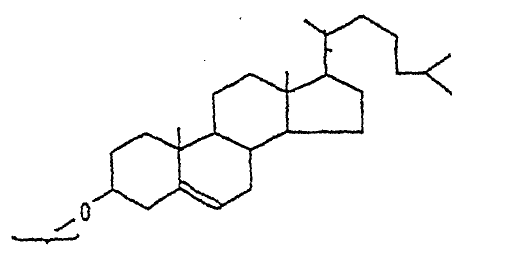 eine Hydroxyl-Gruppe oder irgendeine Oligoribonukleotidyl-Gruppe, die bestimmt wird von einer Strukturformel gemäß dem Derivat nach Anspruch 1, von der irgendein RO, Y₁ und Y₂ entfernt ist;
K gleich irgendeine Phosphoryl-Gruppe, Thiophosphoryl-Gruppe und Alkylaminophosphoryl-Gruppe mit 1 bis 10 Kohlenstoffatomen;
L ein Kohlenwasserstoffrest mit 1 bis 20 Kohlenstoffatomen; und
E eine ganze Zahl von 1 bis 10.

3. Derivat nach Anspruch 1, worin m gleich 25 ist, B₁ bis B₂₇ stehen für die Folge U, U, C, C, C, U, U, U, C, G, C, U, U, U, C, A, A, G, U, C, C, C, U, G, U, U, C und G; Q₁ bis Q₂₆ gleich ein Thioanion; Y₁ und Y₂ gleich OCH₃; und R gleich H (Verbindung XXVII).

4. Derivat nach Anspruch 1, worin m gleich 28 ist, B₁ bis B₃₀ gleich der Folge U, C, C, U, U, C, U, A, G, C, U, C, C, G, C, U, A, G, U, C, A, A, A, A, U, U, U und U; Q₁ bis Q₂₉ jeweils ein Thioanion; Y₁ und Y₂ gleich OCH₃; und R gleich H (Verbindung XXIII).

5. Derivat nach Anspruch 1, worin m gleich 26 ist, B₁ bis B₂₈ gleich der Folge U, U, U, U, A, A, U, U, U, A, U, A, U, U, U, U, U, U, C, U, U, U, C, C, C, C und U; Q₁ bis Q₂₇ jeweils ein Thioanion; Y₁ und Y₂ gleich OCH₃ und R gleich H (Verbindung XXIX).

6. Derivat nach Anspruch 1 mit der Strukturformel

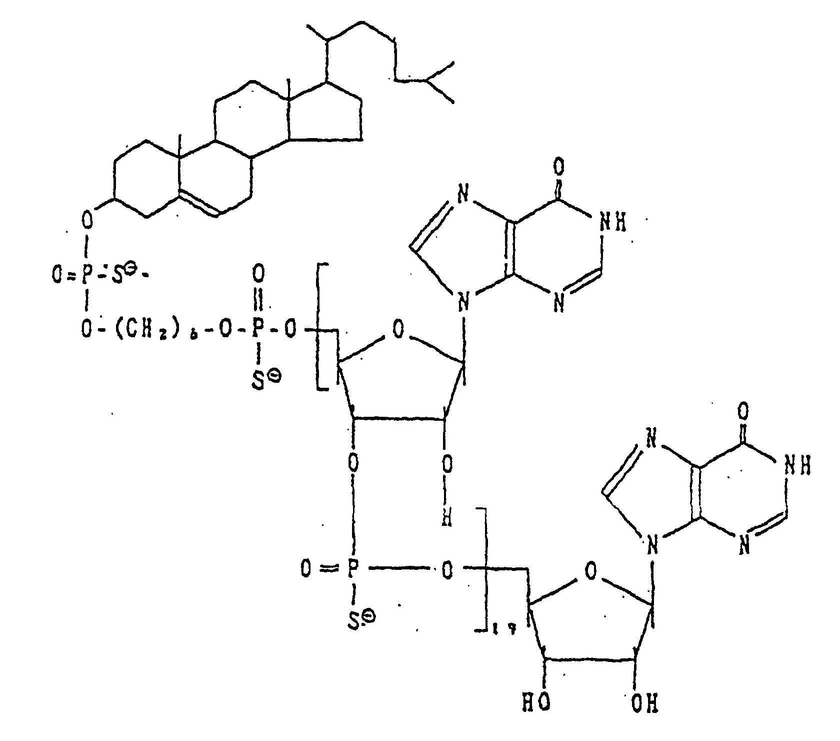

7. Derivat nach Anspruch 1 mit der Strukturformel

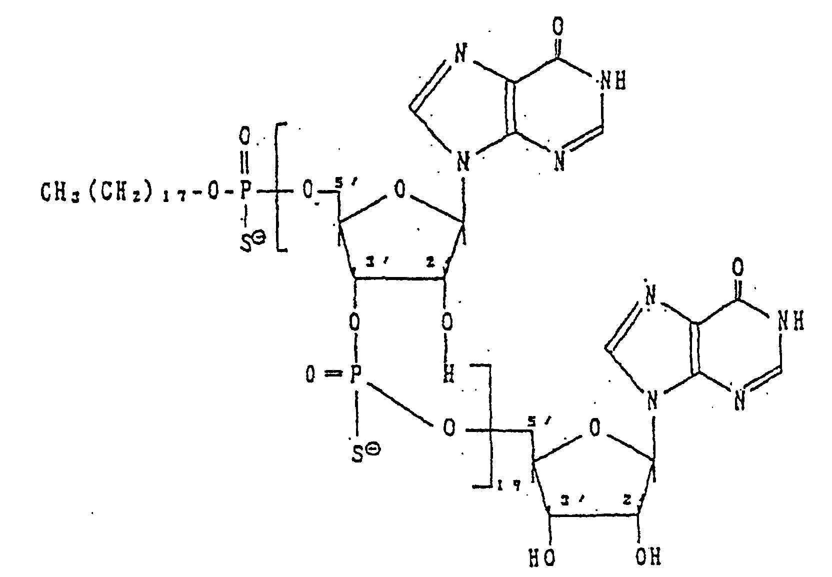

8. Derivat nach Anspruch 1 mit der Strukturformel

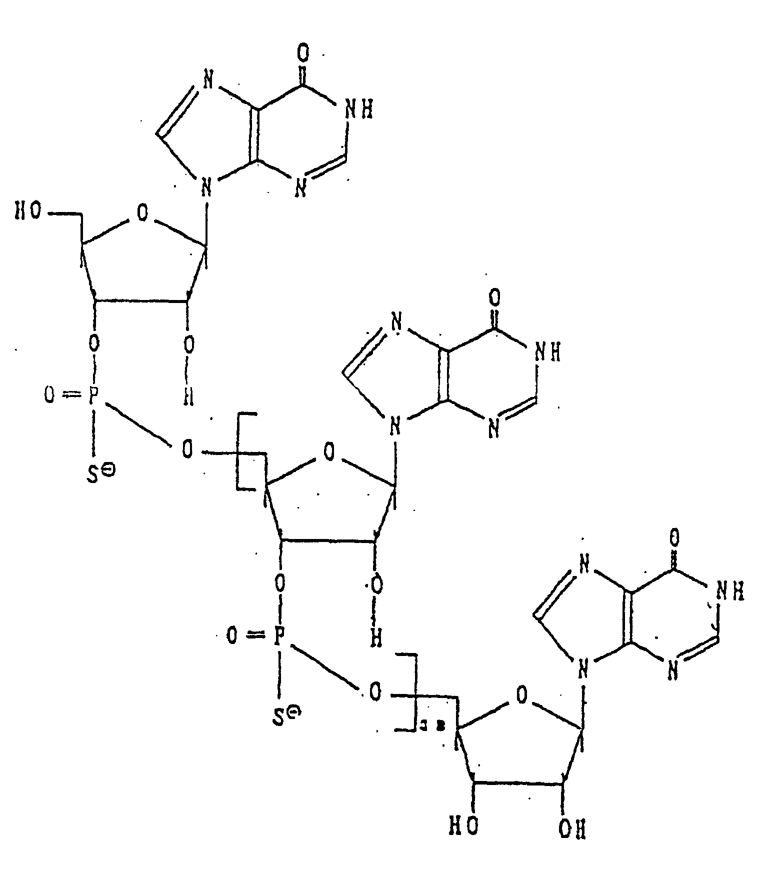

9. Derivat nach Anspruch 1 mit der Strukturformel

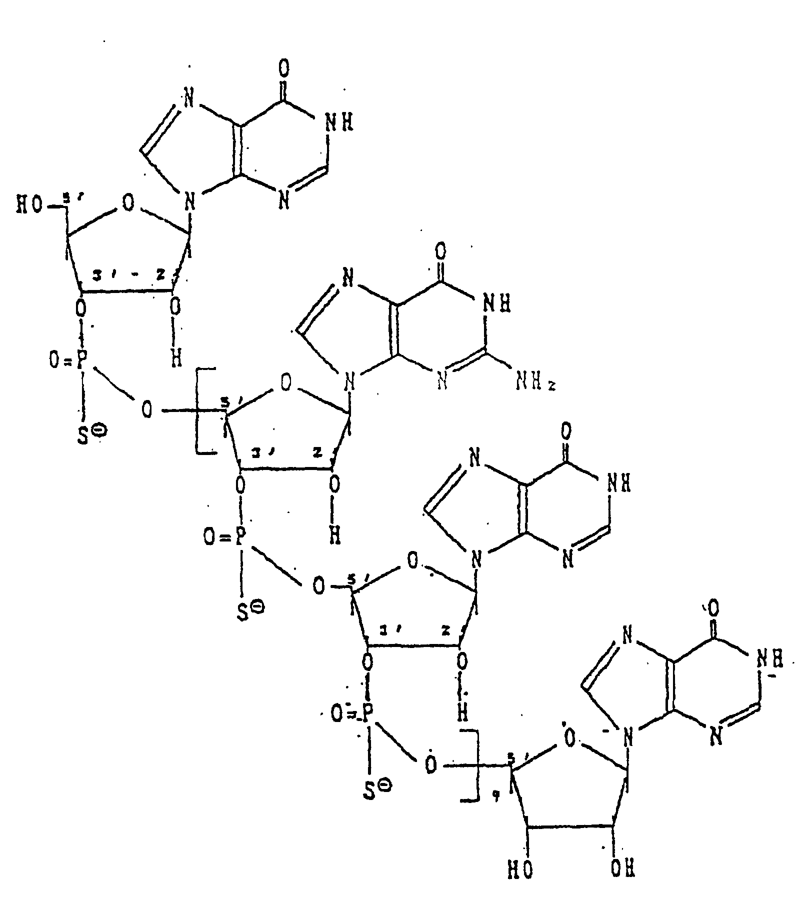

10. Derivat nach Anspruch 1 mit der Strukturformel

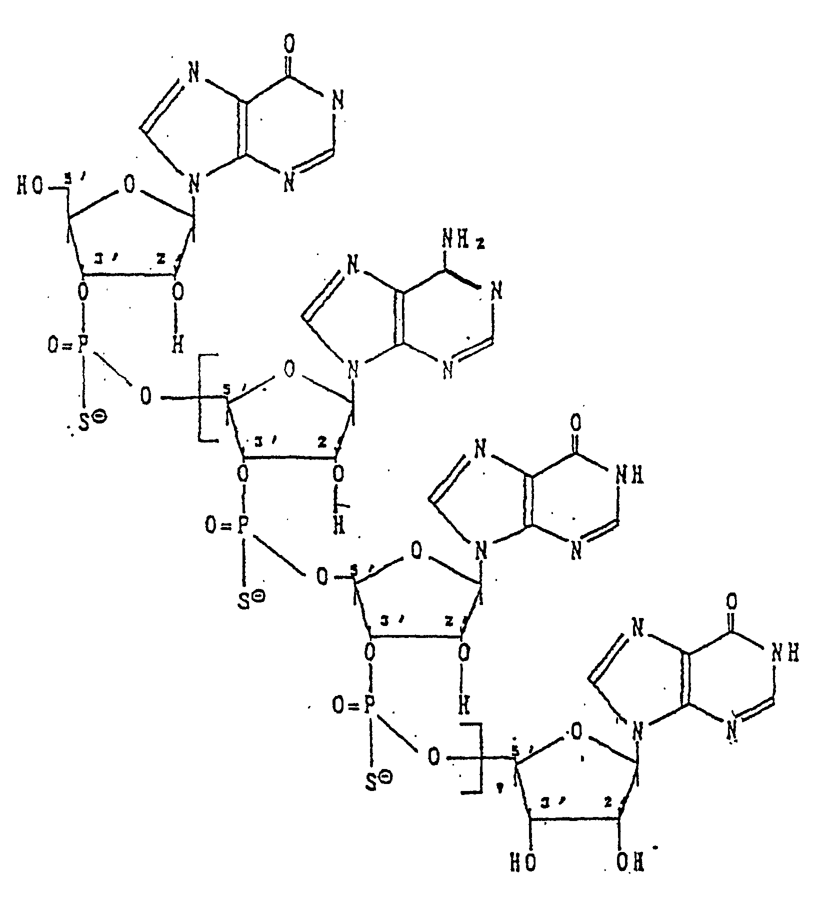

11. Derivat nach Anspruch 1 mit der Strukturformel

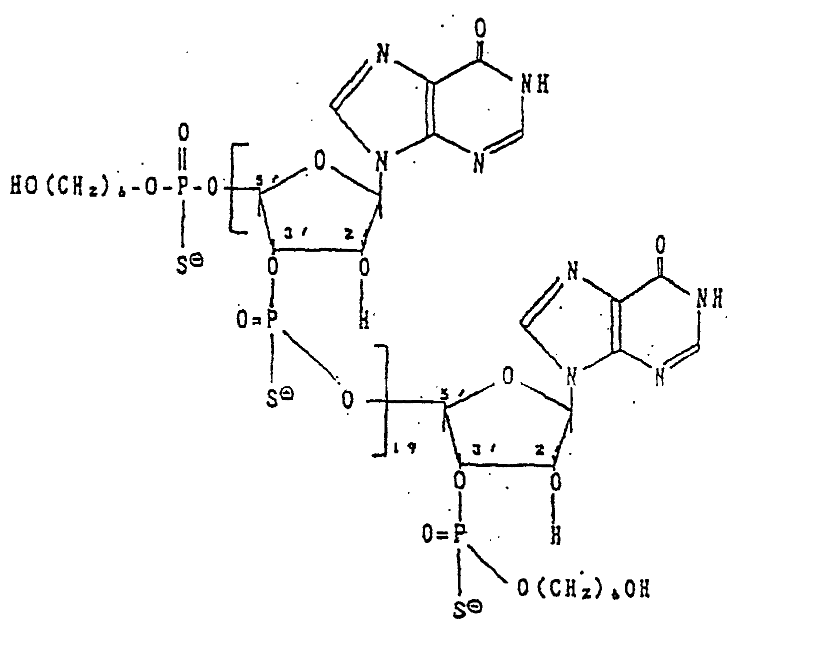

12. Virusmittel gegen AIDS, umfassend als Wirksubstanz mindestens ein Oligoribonukleotid-Derivat nach irgendeinem der Ansprüche 1 bis 11.

13. Oligoribonukleotid-Derivat gemäß der allgemeinen Formel 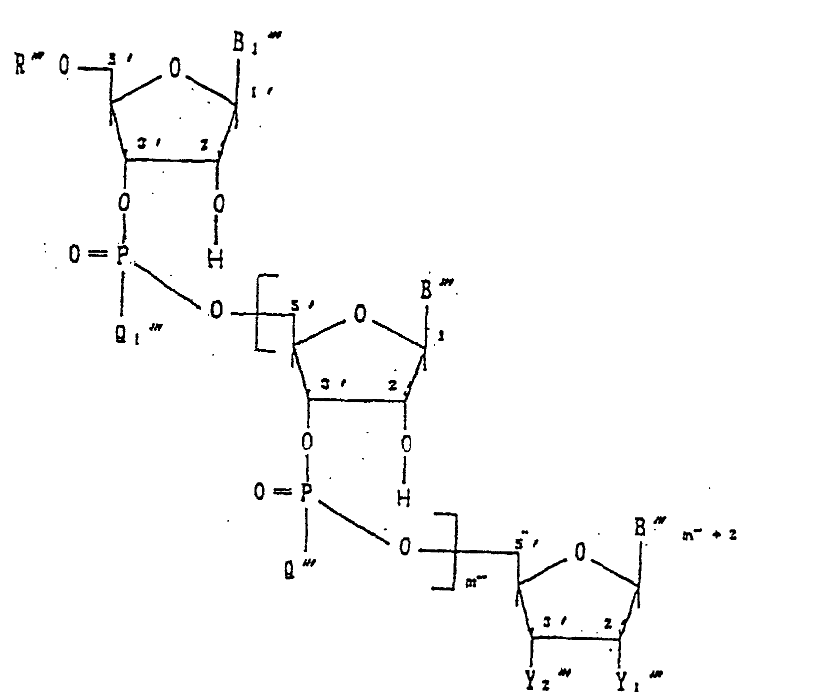 worin ist
m''' eine ganze Zahl von 4 bis 50;
B''' gleich B"'₂, B"'₃,...B'''₍ₘ₊₁₎, und B'''₁ bis B'''_{(m"'+2)}, die gleich oder ungleich sein können, jeweils eine Gruppe aus Hypoxanthin-9-yl, Guanin-9-yl, Adenin-9-yl, Cytosin-1-yl, Uracil-1-yl und Thymin-1-yl, vorausgesetzt, diese sind nicht alle Adenin-9-yl;
Q''' gleich Q"'₂, Q'''₃, ...Q''' _{(m"'+1)}, und Q'''₁ bis Q'''_{(m"'+1)} unabhängig jeweils irgendein Thioanion, Oxoanion, eine Aryloxy-Gruppe, eine Alkylamino-Gruppe, eine Arylamino-Gruppe, eine Alkythio-Gruppe und eine Arylthio-Gruppe, vorausgesetzt, mindestens ein Rest aus Q"'₁ bis Q"'_{(m"'+1)} ist ein Thioanion;
R''' gleich irgendein Wasserstoffatom, eine Acyl-Gruppe mit 1 bis 20 Kohlenstoffatomen und eine Phosphoryl-Gruppe, die wahlfrei einen Substituenten tragen kann;
Y'''₁ und Y'''₂ unabhängig gleich eine Methoxy-Gruppe, eine Acyloxy-Gruppe mit 1 bis 20 Kohlenstoffatomen, eine Alkyloxy-Gruppe, die wahlfrei einen Substituenten tragen kann, eine Hydroxyl-Gruppe, eine Alkylsilyl-Gruppe, ein Wasserstoffatom und eine Phosphoryl-Gruppe, die wahlfrei einen Substituenten tragen kann.

14. Derivat nach Anspruch 13, wobei der Substituent irgendeine Cyanoethyl-Gruppe ist, eine Chlorophenyl-Gruppe und ein Wasserstoffrest mit 1 bis 20 Kohlenstoffatomen.

15. Derivat nach Anspruch 13 mit der Strukturformel

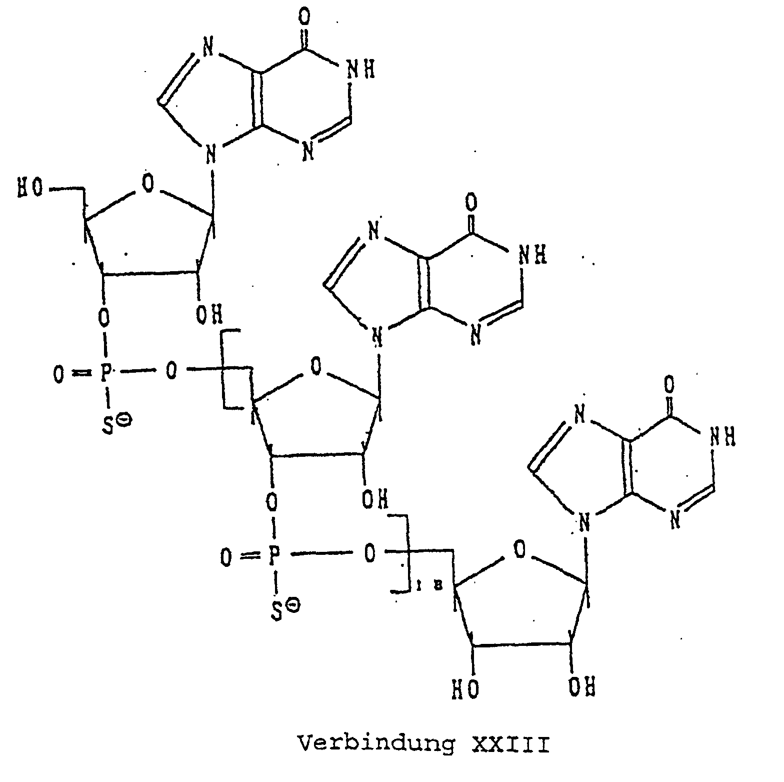

## Revendications

1. Dérivé d'oligoribonucléotide représenté par la formule générale : 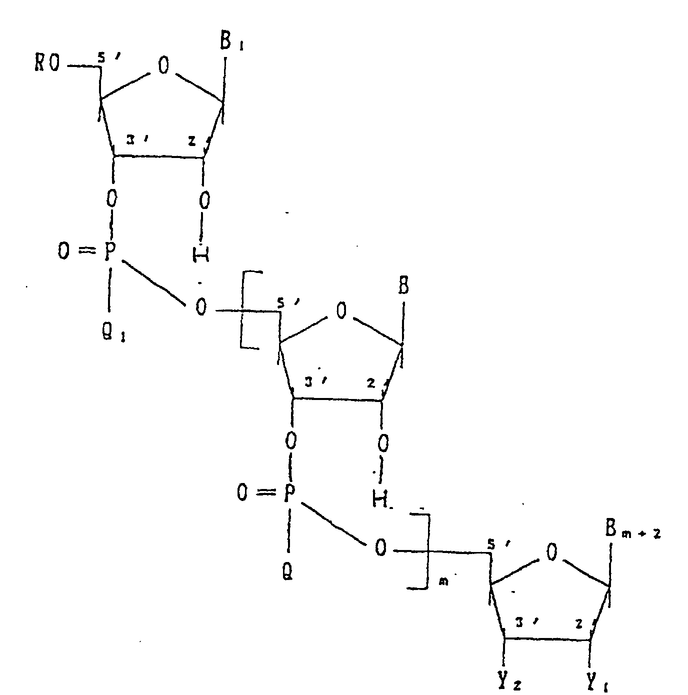 dans laquelle :
m représente un nombre entier de 4 à 50 ;
B représente B₂, B₃, ... B₍ₘ₊₁₎, et B₁ à B₍ₘ₊₂₎, qui peuvent être identiques ou différents, représentent chacun l'un quelconque des résidus hypoxanthine-9-yle, guanine-9-yle, adénine-9-yle, cytosine-1-yle, uracile-1-yle et thymine-1-yle ;
Q représente Q₂, Q₃, ... Q₍ₘ₊₁₎, et Q₁ à Q₍ₘ₊₁₎ représentent, indépendamment, l'une quelconque des entités consistant en un thio-anion, un oxanion, un groupe alkyle, un groupe aryloxy, un groupe alkylamino, un groupe arylamino, un groupe alkylthio et un groupe arylthio (sous réserve qu'au moins un de Q₁ à Q₍ₘ₊₁₎ représente un thioanion) ;
R représente un atome d'hydrogène, un groupe acyle ayant 1 à 20 atomes de carbone, un groupe phosphoryle qui peut porter facultativement un substituant, un groupe thiophosphoryle qui peut porter facultativement un substituant ou un groupe alkylaminophosphoryle qui peut porter facultativement un substituant ;
Y₁ et Y₂ représentent, indépendamment, un groupe méthoxy, un groupe acyloxy ayant 1 à 20 atomes de carbone, un atome d'hydrogène, un groupe hydroxyle, un groupe alkyloxy qui peut porter facultativement un substituant, un groupe phosphoryloxy qui peut porter facultativement un substituant, un groupe thiophosphoryloxy qui peut porter facultativement un substituant et un groupe alkylaminophosphoryloxy qui peut porter facultativement un substituant.

2. Dérivé suivant la revendication 1, dans lequel les substituants sur R, Y et Y₂, qui peuvent être identiques ou différents, représentent chacun l'une quelconque des entités consistant en un groupe cyano-éthyle, un groupe chlorophényle, un groupe monophosphoryle, un groupe pyrophosphoryle, un résidu hydrocarboné ayant 1 à 20 atomes de carbone, un groupe cholestéryle et un groupe répondant à la formule J-(K-O-L)_{E} dans laquelle J représente un groupe : 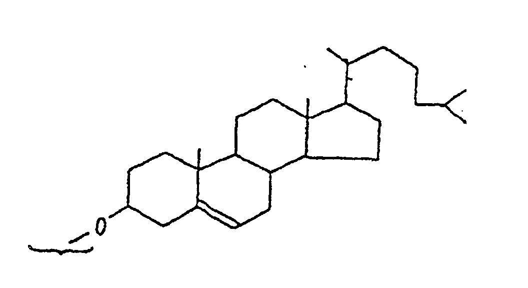 un groupe hydroxyle ou l'un quelconque des groupes oligoribonucléotidyle définis pour la formule structurale dudit dérivé suivant la revendication 1, duquel l'un quelconque des groupes RO, Y₁ et Y₂ est éliminé ;
K représente l'un quelconque des groupes consistant en un groupe phosphoryle, un groupe thiophosphoryle et un groupe alkylaminophosphoryle ayant 1 à 10 atomes de carbone ;
L représente un résidu hydrocarboné ayant 1 à 20 atomes de carbone ; et
E représente un nombre entier de 1 à 10.

3. Dérivé suivant la revendication 1, dans lequel m est égal à 25, B₁ à B₂₇ représentent, en séquence, U, U, C, C, C, U, U, U, C, G, C, U, U, U, C, A, A, G, U, C, C, C, U, G, U, U, C et G ; Q₁ à Q₂₆ représentent chacun un thioanion ; Y₁ et Y₂ représentent un groupe OCH₃ ; et R représente H (composé XXVII).

4. Dérivé suivant la revendication 1, dans lequel m est égal à 28, B₁ à B₃₀ représentent, en séquence, U, C, C, U, U, C, U, A, G, C, U, C, C, G, C, U, A, G, U, C, A, A, A, A, U, U, U et U ; Q₁ à Q₂₉ représentent chacun un thioanion ; Y₁ et Y₂ représentent un groupe OCH₃ ; et R représente H (composé XXIII).

5. Dérivé suivant la revendication 1, dans lequel m est égal à 26, B₁ à B₂₈ représentent, en séquence, U, U, U, U, A, A, U, U, U, A, U, A, U, U, U, U, U, U, C, U, U, U, C, C, C, C, C et U ; Q₁ à Q₂₇ représentent chacun un thioanion ; Y₁ et Y₂ représentent un groupe OCH₃ ; et R représente H (composé XXIX).

6. Dérivé suivant la revendication 1, répondant à la formule structurale :

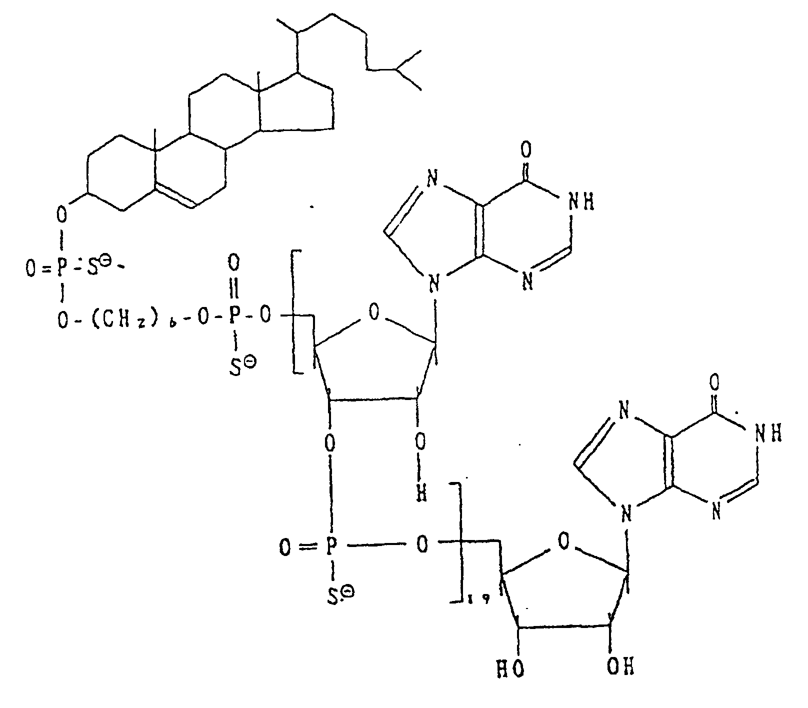

7. Dérivé suivant la revendication 1, répondant à la formule structurale :

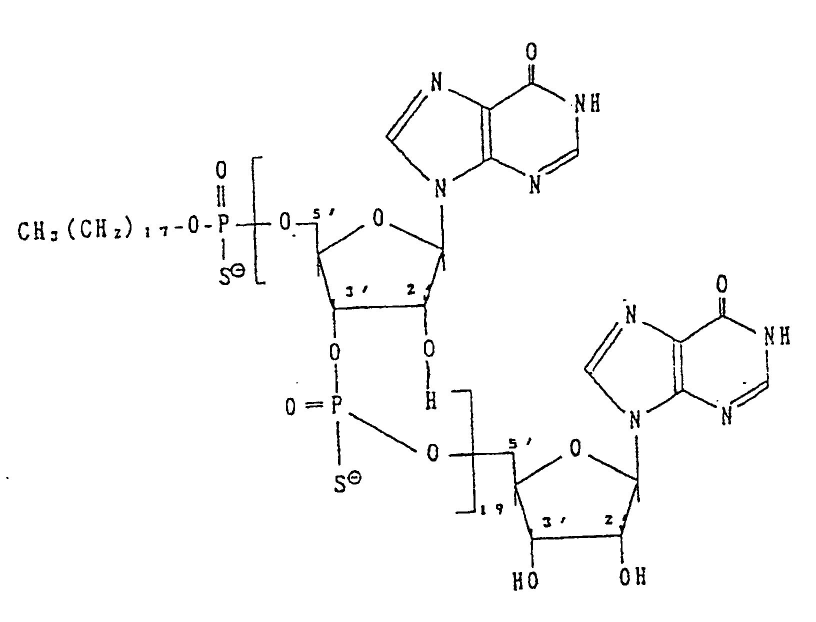

8. Dérivé suivant la revendication 1, répondant à la formule structurale :

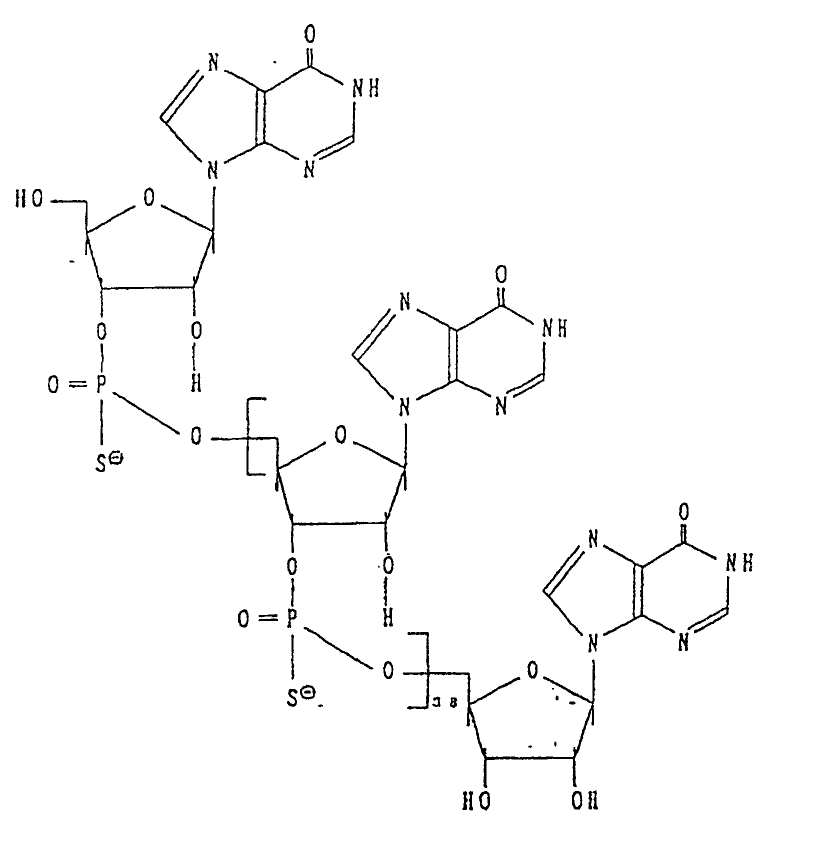

9. Dérivé suivant la revendication 1, répondant à la formule structurale :

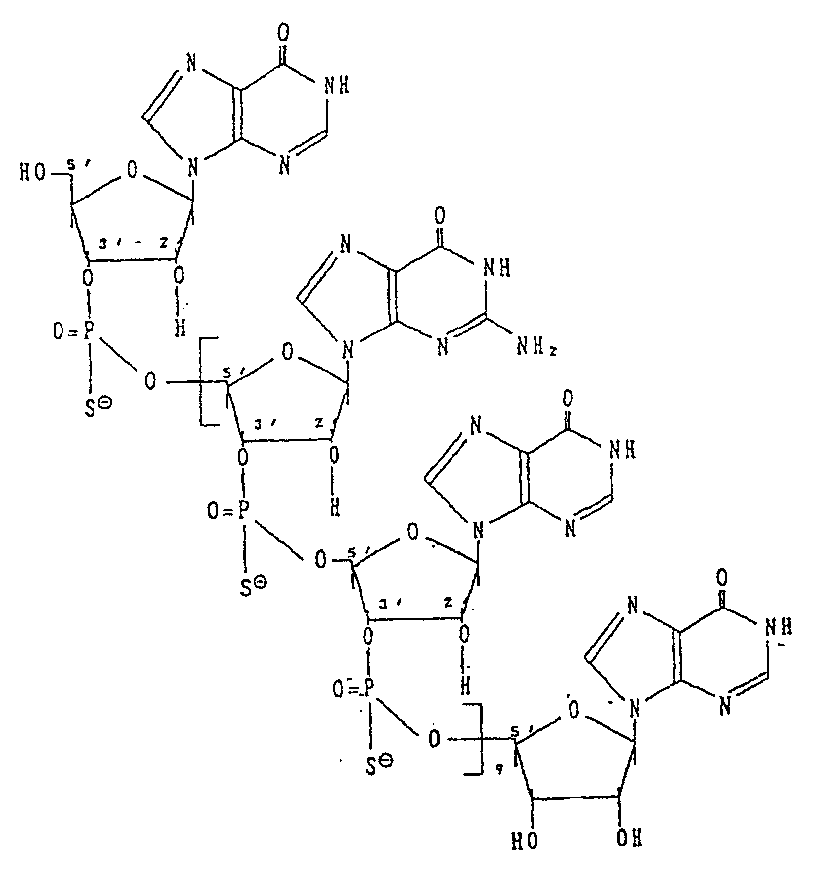

10. Dérivé suivant la revendication 1, répondant à la formule structurale :

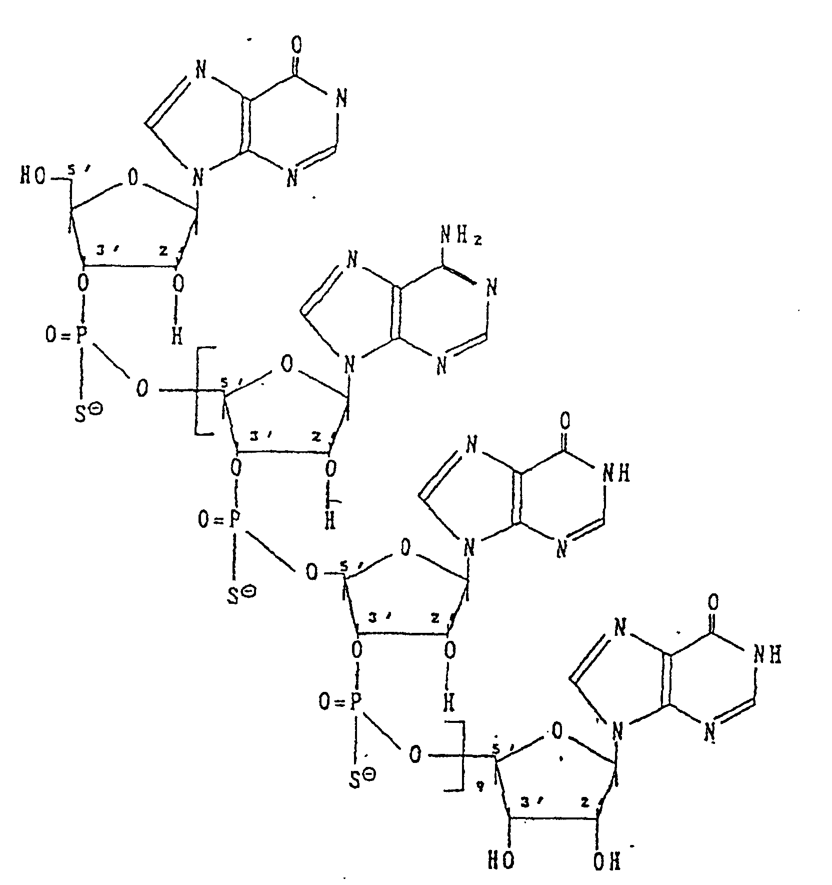

11. Dérivé suivant la revendication 1, répondant à la formule structurale :

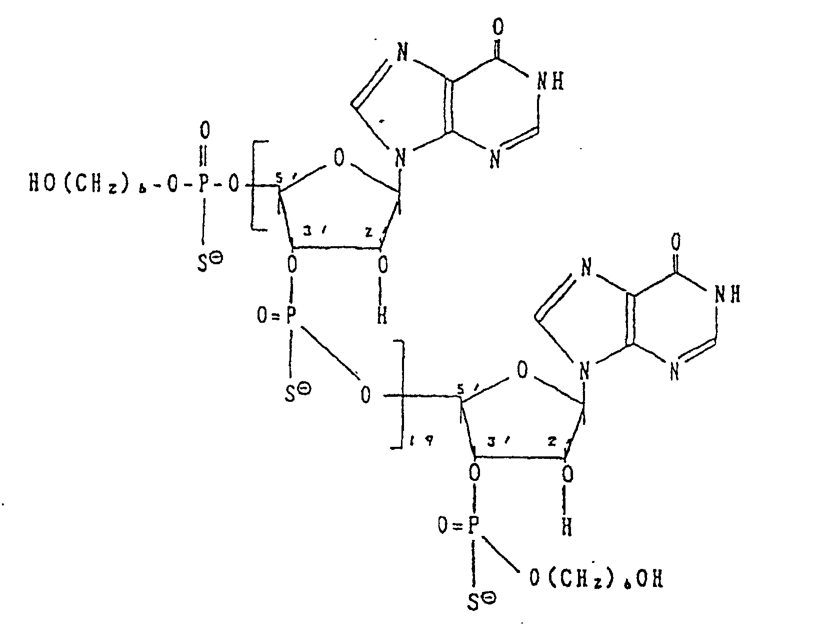

12. Agent viral anti-SIDA comprenant, comme ingrédient efficace, au moins un dérivé d'oligoribonucléotide suivant l'une quelconque des revendications 1 à 11.

13. Dérivé d'oligoribonucléotide représenté par la formule générale : 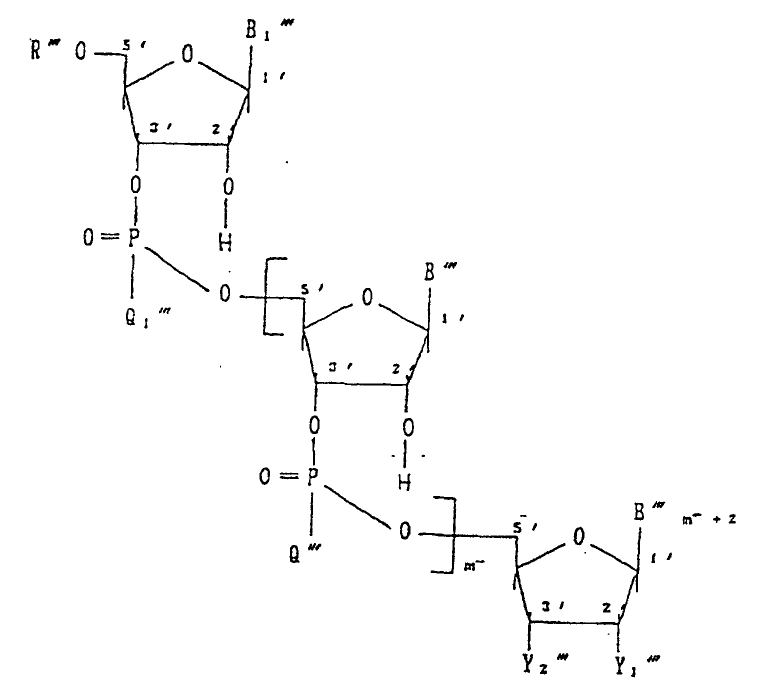 dans laquelle :
m''' représente un nombre entier de 4 à 50 ;
B''' représente B'''₂, B'''₃, ... B'''_{(m'''+1)}, et B'''₁ à B"'_{(m"'+2)}, qui peuvent être identiques ou différents, représentent chacun l'un quelconque des groupes hypoxanthine-9-yle, guanine-9-yle, adénine-9-yle, cytosine-1-yle, uracile-1-yle et thymine-1-yle (sous réserve que tous ces groupes ne représentent pas un groupe adénine-9-yle) ;
Q'' ' représente Q"'₂, Q'''₃, ... Q'''_{(m'''+1)}, et Q'''₁ à Q'''_{(m'''+1)} représentent, indépendamment, l'une quelconque des entités consistant en un thio-anion, un oxanion, un groupe aryloxy, un groupe alkylamino, un groupe arylamino, un groupe alkylthio et un groupe arylthio (sous réserve qu'au moins un de Q'''₁ 1 à Q'''_{(m'''+1)} représente un thioanion ;
R''' représente l'une quelconque des entités consistant en un atome d'hydrogène, un groupe acyle ayant 1 à 20 atomes de carbone et un groupe phosphoryle qui peut porter facultativement un substituant ;
Y" ' ₁ et Y'''₂ représentent, indépendamment, l'un quelconque des groupes consistant en un groupe méthoxy, un groupe acyloxy ayant 1 à 20 atomes de carbone, un groupe alkyloxy qui peut porter facultativement un substituant, un groupe hydroxyle, un groupe alkylsilyle, un atome d'hydrogène et un groupe phosphoryle qui peut porter facultativement un substituant.

14. Dérivé suivant la revendication 13, dans lequel le substituant consistant en l'une quelconque des entités consistant en un groupe cyano-éthyle, un groupe chlorophényle et un résidu hydrocarboné ayant 1 à 20 atomes de carbone.

15. Dérivé suivant la revendication 13, répondant à la formule structurale :
